# EUROPEAN PATENT APPLICATION

(11) **EP 4 144 758 A1**
(43) Date of publication of application: **08.03.2023**
(21) Application number: 21793484.3
(22) Date of filing: 21.04.2021
(51) Int. Cl.: C07K 16/28, A61K 39/395, A61P 35/00

(54) **SINGLE VARIABLE DOMAIN ANTIBODY TARGETING HUMAN PROGRAMMED DEATH LIGAND 1 (PD-L1) AND DERIVATIVE THEREOF**

(30) Priority: 22.04.2020 CN 202010324761
(71) Applicant: Mabwell (Shanghai) Bioscience Co., Ltd., Shanghai 201210 (CN)
(72) Inventor: WANG, Shuang, Shanghai 201210 (CN); ZENG, Dadi, Shanghai 201210 (CN); JIAO, Shasha, Shanghai 201210 (CN); ZHANG, Chang, Shanghai 201210 (CN); WANG, Rongjuan, Shanghai 201210 (CN)
(74) Representative: Latscha Schöllhorn Partner AG
(86) International application number: PCT/CN2021/088674
(87) International publication number: WO 2021/213435

(57) **Abstract**

Provided are a single variable domain antibody targeting a human programmed death ligand 1 (PD-L1) and a derivative thereof. A single variable domain antibody 2-2F2 specific for human PD-L1 is identified and obtained by extracting PBMCs from a camel immunized with human PD-L1, constructing a phage surface displayed VHH antibody library, and screening said VHH antibody library. On said basis, a chimeric antibody chF2 and a humanized modified antibody hzF2 variant are prepared. The hzF2 variant possesses affinity comparable or even superior to that of the original single variable domain antibody 2-2F2, can block the binding of PD-1 to PD-L1 *in vitro,* and can inhibit tumor growth in an *in vivo* experiment on tumor-bearing mice.

## Description

The present application claims the priority right of Chinese Patent Application for Invention No. CN 202010324761.8, filed on April 22, 2020, which is hereby incorporated by reference in its entirety.

### Technical Field

The present invention relates to the field of antibody drugs. In particular, the present invention relates to a single variable domain antibody targeting human programmed death ligand 1 (PD-L1), a protein derived therefrom and a use thereof for preparing drugs, particularly a use for the treatment and/or prevention, or diagnosis of PD-L1 related diseases such as tumors.

### Background Art

PD-1 and its ligand PD-L1 are important targets of tumor immunity. PD-1 and PD-L1 are a pair of immunosuppressive molecules, which are important components of the immune system to prevent overreaction of autoimmune response. Activation of the pathway of PD-1 and PD-L1 has the functions of inhibiting tumor immune responses, inducing tumor-specific T cell apoptosis, which are closely related to tumor development. PD-1 (CD279) is a type I transmembrane protein, a member of the immunoglobulin superfamily, mainly expressed on activated CD4+ T cells, CD8+ T cells and B cells and other immune cells. Its ligand PD-L1 (also known as B7-H1, CD274) belongs to a member of the B7 family and is highly expressed on tumor-infiltrating immune cells (TIC) and a variety of malignant tumor cells, such as malignant melanoma, non-small cell lung cancer, head and neck squamous cancer etc.. The use of a monoclonal antibody to block PD-1 and PD-L1 pathway in the treatment of tumors has shown good clinical efficacy and safety. Many antibody drugs have been approved for marketing, and the indications include many malignant tumors such as melanoma, non-small cell lung cancer, advanced renal cell carcinoma and the like. Meanwhile, many ongoing clinical trials try to develop more new indications.

Although PD-1/PD-L1 monoclonal antibodies have shown good therapeutic effects in the clinical treatments of various malignant tumors, there are problems such as large dosage and low overall response rate. The main causes include low expression of PD-L1 and depletion of T cells in the tumor microenvironment and the like. Therefore, it is still necessary to further deeply develop the PD-1/PD-L1 targets and develop therapeutic drugs with better clinical effects. With respect to drug development for the targets, the following ways may increase the benefits of clinical patients. (1) Further developing antibody molecules having higher affinity and better activity; (2) Providing bispecific antibodies or analogs based on the targets; (3) Preliminary studies have shown that the response rate of PD-L1 positive tumor patients to a PD-L1 inhibitor is much higher than that of PD-L1 negative tumor patients, so effective biomarkers are required to predict PD-L1 positive tumor patients or to screen patients in advance, in order to reduce treatment costs and potential serious adverse reactions; (4) To improve the response rate, drug combination has become a trend of tumor immunotherapy, such as combination with other tumor immune drugs, combination with targeting drugs, chemotherapy or radiotherapy.

A single variable domain antibody is currently the smallest antibody molecule, and its molecular weight is 1/10 of that of a conventional antibody. It is originally discovered in camel blood by Belgian scientist Hamers, R. It is a class of engineered antibody products that has attracted much attention. In addition to having the antigenic reactivity of a monoclonal antibody, a single variable domain antibody also possesses some unique functional properties, such as small molecular weight, strong stability, good solubility, easy expression, strong targeting activity, and simplicity of being humanized. In particular, a single variable domain antibody is suitable for the development of bispecific/multispecific therapeutic antibodies and for the development of therapies such as Car-T/M/NK. At present, the developments of single variable domain antibodies and/or bispecific/multispecific antibodies based on single variable domain antibodies have become research and development hotspots. Internationally, Ablynx has made extensive layouts in the field of single variable domain antibodies. Caplacizumab, developed by the company, was approved by the FDA in February 2019 for the treatment of a rare disease of acquired thrombotic thrombocytopenic purpura (aTTP). Acquired thrombotic thrombocytopenic purpura is characterized by excessive blood clotting in small blood vessels, and Caplacizumab is the first drug approved for the disease. Caplacizumab is also the first drug targeting von willebrand factor (vWF), with vWF being a key protein in the blood coagulation cascade. At the same time, Caplacizumab is also the first single variable domain antibody approved by the FDA. The approval of Caplacizumab is a landmark event for the single variable domain antibody drug field to officially enter the stage of human disease therapy. There are also companies in China that are actively developing single variable domain antibodies, including Shenzhen Guochuang Single Variable Domain Antibody Technology Co., Ltd., Shenzhen Prekin Biopharmaceutical Co., Ltd., Suzhou Boshengji (Anke) Company, and Suzhou Corning Jerry Company, etc.. Among them, Corning Jerry Company is domestically the first to enter the field of single variable domain antibodies. The PD-L1 single variable domain antibody (KN035, subcutaneous administration) developed by Corning Jerry Company was approved by CFDA and FDA to enter clinical trials in 2016, and obtained the clinical approval from the Japan Pharmaceuticals and Medical Devices Agency (PMDA) at the end of June, 2017. KN035 is the world's first PD-L1 single variable domain antibody.

At present, PD-1/PD-L1 monoclonal antibodies have shown good therapeutic effects in the clinical treatment of various malignant tumors, but there are problems such as large dosage and low overall response rate. The main causes include low expression of PD-L1 and depletion of T cells in the tumor microenvironment and the like. Therefore, it is necessary to discover new anti-PD-L1 antibody drugs. With respect to the development of candidate molecules, antibody molecules with better therapeutic effect may be obtained through the following ways: (1) Further developing antibody molecules having higher affinity and better activity; (2) Providing bispecific antibodies or analogs based on the target; (3) Developing more effective diagnostic antibodies, to predict PD-L1-positive tumor patients or screen patients in advance by detecting PD-L1 expression, in order to reduce treatment costs and potential serious adverse reactions. Camel-derived single variable domain antibodies are expected to be used to efficiently solve the above problems in view of their unique properties.

### Summary of the invention

To solve the above problems, the present disclosure provides a single variable domain antibody targeting human programmed death ligand 1 (PD-L1), and derivatives thereof. A phage surface-displayed VHH antibody library is constructed by extracting PBMC from human PD-L1 immunized camels, and an anti-human PD-L1-specific single variable domain antibody 2-2F2 is obtained by screening and identification. On this basis, a chimeric antibody chF2 and a humanized modified antibody hzF2 variant are prepared. The hzF2 variant possesses affinity comparable or even superior to that of the original single variable domain antibody 2-2F2, can block the binding of PD-1 to PD-L1 *in vitro,* and can inhibit tumor growth in an *in vivo* experiment on tumor-bearing mice. In particular:
In a first aspect, the present invention provides an anti-PD-L1 single variable domain antibody, characterized in that CDR1-CDR3 in a variable region of the single variable domain antibody are shown as SEQ ID NOs: 43-45 respectively.

Further, the anti-PD-L1 single variable domain antibody of the present invention is characterized in that the single variable domain antibody has no constant region or has 1-3 heavy chain constant regions.

Further, the anti-PD-L1 single variable domain antibody of the present invention is characterized in that the amino acid sequence of the variable region of the single variable domain antibody is shown as SEQ ID NO: 1.

In the second aspect, the present invention provides an anti-PD-L1 single variable domain antibody, characterized in that the single variable domain antibody is a human-camel chimeric single variable domain antibody, comprising the variable region of the single variable domain antibody according to the first aspect of the present invention and human heavy chain constant regions.

Further, the anti-PD-L1 single variable domain antibody of the present invention is characterized in that the chimeric single variable domain antibody has the amino acid sequence shown as SEQ ID NO:3.

In a third aspect, the present invention provides an anti-PD-L1 single variable domain antibody, characterized in that the single variable domain antibody is humanized, and the variable region of the single variable domain antibody is obtained by humanizing the variable region of the single variable domain according to the first aspect of the present invention.

Further, the anti-PD-L1 single variable domain antibody of the present invention is characterized in that the variable region of the single variable domain antibody has the amino acid sequence shown as SEQ ID NO:7.

Further, the anti-PD-L1 single variable domain antibody of the present invention is characterized in that the single variable domain antibody has the amino acid sequence shown as SEQ ID NO:9.

In a fourth aspect, the present invention provides an anti-PD-L1 single variable domain antibody, characterized in that the single variable domain antibody is a mutated anti-PD-L1 humanized single variable domain antibody, which is produced by mutating CDRs in the variable region of anti-PD-L1 single variable domain antibody according to the third aspect of the present invention by 1, 2, 3 or 4 amino acid residues; and the mutated anti-PD-L1 humanized single variable domain antibody at least partially retains the specific binding ability to PD-L1.

Further, the anti-PD-L1 single variable domain antibody of the present invention is characterized in that the variable region thereof is selected from the group consisting of SEQ ID NOs: 11-26.

In a fifth aspect, the present invention provides a composition comprising one or more anti-PD-L1s selected from the group consisting of the anti-PD-L1 single variable domain antibodies according to any of the first to the fourth aspects of the present invention.

Further, the composition of the present invention is characterized in that it further comprises a pharmaceutically acceptable carrier, and is used as a pharmaceutical composition, preferably the pharmaceutical composition is in the form of a liquid formulation, an injection formulation, or a powder injection formulation.

In a sixth aspect, the present invention provides the use of an antibody or its fragment for the manufacture of a medicament for the treatment of abnormal proliferative diseases, characterized in that the antibody is selected from the group consisting of the anti-PD-L1 single variable domain antibody according to any of the first to the fourth aspects of the present invention.

Further, the use of the present invention is characterized in that the abnormal proliferative diseases comprise tumors, preferably melanoma, non-small cell lung cancer, head and neck squamous carcinoma, kidney cancer, colon cancer and the like.

In a seventh aspect, the present invention provides the use of an antibody or its fragment, characterized in that the antibody is selected from the group consisting of the anti-PD-L1 single variable domain antibody according to any of the first to the fourth aspects of the present invention, for the manufacture of a multispecific antibody or a targeted antibody-drug.

In an eighth aspect, the present invention provides a polynucleotide encoding the anti-PD-L1 single variable domain antibody according to any of the first to the fourth aspects of the present invention.

In a ninth aspect, the present invention provides a vector comprising the polynucleotide according to the eighth aspect of the present invention.

In a tenth aspect, the present invention provides a host cell comprising the polynucleotide according to the seventh aspect of the present invention or the vector according to the eighth aspect of the present invention.

In an eleventh aspect, the present invention provides a method for preparing an anti-PD-L1 single variable domain antibody, comprising the steps of:
(1) Culturing the host cell according to the tenth aspect of the present invention under conditions suitable for expressing the recombinant anti-PD-L1 single variable domain antibody;
(2) Isolating and purifying the anti-PD-L1 single variable domain antibody from the cell culture.

In a twelfth aspect, the present invention provides a method for preventing or treating abnormal proliferative diseases, characterized in that administering to a subject in need thereof an effective amount of the anti-PD-L1 single variable domain antibody according to any one of the foregoing, the composition according to any one of the foregoing, the multispecific antibody or the targeted antibody-drug in the use according to the aforementioned sixth aspect.

Further, the method of the present invention is characterized in that: the abnormal proliferative diseases comprise tumors, particularly tumors associated with PD-1/PD-L1 signaling pathway.

Further, the method of the present invention is characterized in that the tumors comprise melanoma, non-small cell lung cancer, head and neck squamous carcinoma, kidney cancer, colon cancer and the like.

In a thirteenth aspect, the present invention provides a method for diagnosing or evaluating the development and progress of abnormal proliferative diseases in a subject, characterized in that contacting a sample from the subject to be detected with the anti-PD-L1 single variable domain antibody according to any one of the foregoing, the composition according to any one of the foregoing, the multispecific antibody or the targeted antibody-drug in the use according to the aforementioned sixth aspect.

Further, the method of the present invention is characterized in that the abnormal proliferative diseases comprise tumors, particularly tumors related to PD-1/PD-L1 signaling pathway.

Further, the method of the present invention is characterized in that the tumors comprise melanoma, non-small cell lung cancer, head and neck squamous carcinoma, kidney cancer, colon cancer and the like.

In a fourteenth aspect, the present invention provides a method for predicting or evaluating the therapeutic effect of a PD-1/PD-L1 antagonist on a subject suffering from an abnormal proliferative disease, characterized in that detecting the expression status of PD-L1 in the subject by using an agent selected from the group consisting of the anti-PD-L1 single variable domain antibody according to any one of the foregoing, the composition according to any one of the foregoing, the multispecific antibody or the targeted antibody-drug in the use according to the aforementioned sixth aspect.

Further, the method of the present invention is characterized in that the abnormal proliferative diseases comprise tumors, particularly tumors related to PD-1/PD-L1 signaling pathway.

Further, the method of the present invention is characterized in that the tumors comprise melanoma, non-small cell lung cancer, head and neck squamous carcinoma, kidney cancer, colon cancer and the like.

Unless otherwise indicated, regardless of being used herein to refer to heavy chain antibodies or to conventional 4-chain antibodies, the term "immunoglobulin sequence" is used as a generic term to include full-size antibodies, individual chains thereof, and all portions, domains or fragments thereof (including, but not limited to, antigen binding domains or fragments such as VHH domains or VH/VL domains, respectively). Furthermore, the term "sequence" as used herein (e.g., used in terms such as "immunoglobulin sequence", "antibody sequence", "variable domain sequence", "VHH sequence" or "protein sequence") generally should be understood to comprise both the relevant amino acid sequence and the nucleic acid or nucleotide sequence encoding thereof, unless the context requires a more restrictive interpretation.

An immunoglobulin single variable domain can act as a "binding unit", "binding domain" or "construction unit" of a polypeptide (these terms can be used interchangeably), and be used for preparing a polypeptide comprising one or more additional immunoglobulin single variable domains as the binding unit (i.e., against the same or different epitopes on the same target and/or against one or more different targets).

The term "immunoglobulin single variable domain" ("ISVD"), which can be used interchangeably with "single variable domain" ("SVD"), defines a molecule in which the antigen binding site is present on a single immunoglobulin domain and which consists of a single immunoglobulin domain. This makes the immunoglobulin single variable domain different from a "conventional" immunoglobulin or fragments thereof, in which two domains, especially two variable domains in the "conventional" immunoglobulin, interact to form an antigen binding site. Typically, in conventional immunoglobulins, the heavy chain variable domain (VH) and light chain variable domain (VL) interact to form the antigen binding site. Under the circumstances, the complementarity determining regions (CDRs) from both VH and VL would favor the antigen binding site, i.e. a total of 6 CDRs would be involved in the formation of the antigen binding site.

In contrast, the binding site of an immunoglobulin single variable domain is formed by a single VH or VL domain. Thus, the antigen binding site of an immunoglobulin single variable domain is formed by no more than three CDRs.

The terms "immunoglobulin single variable domain" and "single variable domain" thus are not comprised in conventional immunoglobulins or fragments thereof that require at least two variable domains to interact to form an antigen binding site. However, these terms are comprised in fragments of conventional immunoglobulins, in which an antigen binding site is formed by a single variable domain.

Typically, a single variable domain will be an amino acid sequence consisting substantially of 4 framework regions (FR1 to FR4, respectively) and 3 complementarity determining regions (CDR1 to CDR3, respectively). Such a single variable domain and fragments are most preferable such that they comprise immunoglobulin folds or are capable of forming immunoglobulin folds under suitable conditions. Thus, a single variable domain may, for example, comprise a light chain variable domain sequence (e.g., a VL sequence) or a suitable fragment thereof; or a heavy chain variable domain sequence (e.g., a VH sequence or a VHH sequence) or a suitable fragment thereof, provided that they are capable of forming a single antigen-binding unit (i.e., a functional antigen-binding unit consisting essentially of a single variable domain such that the single antigen-binding unit does not need to interact with another variable domain to form a functional antigen binding unit. For example, the variable domains found in e.g. conventional antibodies and scFv fragments that require VH/VL interaction thus interact with another variable domain to form a functional antigen binding domain is the latter).

In one embodiment of the invention, the immunoglobulin single variable domain is a light chain variable domain sequence (e.g., a VL sequence) or a heavy chain variable domain sequence (e.g., a VH sequence); more specifically, the immunoglobulin single variable domain can be a heavy chain variable domain sequence derived from a conventional four-chain antibody or a heavy chain variable domain sequence derived from a heavy chain antibody.

For example, a single variable domain or an immunoglobulin single variable domain (or amino acids suitable for use as an immunoglobulin single variable domain) can be a (single) domain antibody (or amino acids suitable for use as a (single) domain antibody), "dAbs" or dAbs (or amino acids suitable for use as dAbs) or nanobodies (as defined herein, and including but not limited to VHHs); other single variable domains, or any suitable fragments of any of them.

For a general description of (single) domain antibodies, reference is also made to the prior art cited herein and to EP0368684. For the term "dAb", see e.g. Ward et al., 1989 (Nature 341: 544-546), see Holt et al., 2003 (Trends Biotechnol., 21: 484-490); and see e.g. WO 04/068820, WO 06/030220, WO06/003388, WO 06/059108, WO 07/049017, WO 07/085815, and other published patent applications of Domantis Ltd. It should also be noted that, single variable domains may be derived from certain species of sharks (e.g. so-called "IgNAR domains", see e.g. WO 05 /18629), although they are less preferable in the context of the present invention as not of mammalian origin.

In particular, the immunoglobulin single variable domain may be NANOBODY^{®} (as defined herein) or a suitable fragment thereof. [Note: NANOBODY^{®}, NANOBODIES^{®}, NANOCLONE^{®} are registered trademarks of Ablynx N.V]. For a general description of nanobodies, reference is made to the further description below, as well as to the prior art cited herein, for example, described in such as WO 08/020079 (page 16).

For a further description of VHHs and nanobodies, reference is made to the review article by Muyldermans 2001 (Reviews in Molecular Biotechnology 74: 277-302) and to the following patent applications mentioned as general background art: WO 94/04678, WO 95/04079, and WO 96/34103 of VrijeUniversiteit Brussel; WO 94/25591, WO 99/37681, WO 00/40968, WO 00/43507, WO00/65057, WO 01/40310, WO 01/44301, EP 1134231, and WO 02/48193 of Unilever; WO 97/49805, WO 01/21817, WO 03/035694, WO 03/054016, and WO 03/055527 of Vlaams Instituutvoor Biotechnologie (VIB); WO 03/050531 of Algonomics N.V, and Ebolinx, Inc.; WO 01/90190 of National Research Council of Canada; WO 03/025020 of Institute of Antibodies; and WO 04/041867, WO 04/041862, WO 04/041865, WO 04/041863, WO 04/062551, WO 05/044858, WO06/40153, WO 06/079372, WO 06/122786, WO 06/122787, and WO 06/122825 of Ebolinx, Inc.; and other published patent applications of Ebolinx, Inc.. Reference is also made to other prior art mentioned in these applications, and in particular to the list of references mentioned on pages 41-43 of International Application WO 06/040153, which list and references are incorporated herein by reference. As described in these references, nanobodies (especially VHH sequences and a part of humanized nanobodies) may be characterized, inter alia, by the presence of one or more "marker residues" in one or more framework sequences. Further descriptions of nanobodies, including humanized and/or camelized nanobodies, as well as other modifications, parts or fragments, derivatives thereof or "nanobody fusions", multivalent constructs (some non-limiting examples comprising linker sequences) and different modifications to increase the half-life of nanobodies as well as their preparation, can be found, for example, in WO 08/101985 and WO 08/142164.

Thus, in the sense of the present invention, the term "immunoglobulin single variable domain" or "single variable domain" comprises polypeptides derived from non-human sources, preferably camelid, preferably camelid heavy chain antibodies. As described above, they can be humanized. In addition, the term comprises polypeptides derived from non-camelid sources such as mice or humans that have been "camelized", e.g., described in Davies and Riechmann 1994 (FEBS 339: 285-290), 1995 (Biotechnol. 13: 475- 479), 1996 (Prot. Eng. 9:531-537), and Riechmann and Muyldermans 1999 (J. Immunol. Methods 231: 25-38).

The term "immunoglobulin single variable domain" comprises immunoglobulin sequences of different origins, including mouse, rat, rabbit, donkey, human and camelid immunoglobulin sequences. It also comprises fully human, humanized or chimeric immunoglobulin sequences. For example, it comprises camelid immunoglobulin sequences and humanized camelid immunoglobulin sequences, or camelized immunoglobulin single variable domains, such as the camelized dAbs described by Ward et al., 1989 (See e.g. WO 94/04678 and Davies and Riechmann 1994, 1995 and 1996) and camelized VH.

Likewise, such immunoglobulin single variable domains may be derived from any suitable source in any suitable manner, and can, for example, be naturally occurring VHH sequences (i.e., from a suitable camelid species) or synthetic or semi-synthetic amino acid sequences, including but not limited to partially or fully "humanized" VHH, "camelized" immunoglobulin sequences (and especially camelized VH), as well as nanobodies and/or VHHs obtained by the following techniques: such as affinity maturation (e.g., starting from synthetic, random or naturally occurring immunoglobulin sequences such as VHH sequences), CDR grafting, veneering, combining fragments derived from different immunoglobulin sequences, PCR assembly using overlapping primers, and similar techniques known to those skilled in the art for engineering immunoglobulin sequences; or any suitable combination of any of the foregoing.

The amino acid sequence and structure of an immunoglobulin single variable domain may be considered as, but is not limited to, consisting of four framework regions or "FRs", which in the art and herein are referred to as "framework region 1" or "FR1"; "framework region 2" or "FR2"; "framework region 3" or "FR3"; and "framework region 4" or "FR4", respectively; interspersed by 3 complementarity determining regions or "CDRs", which are referred to in the art as "complementarity determining region 1" or "CDR1"; "complementarity determining region 2" or "CDR2"; and "complementarity determining region 3" or "CDR3", respectively.

The total number of amino acid residues in an immunoglobulin single variable domain may range from 110-120, preferably 112-115, and most preferably 113.

As further described in paragraph q) on pages 58 and 59 of WO 08/020079 (incorporated herein by reference), numbering of amino acid residues in an immunoglobulin single variable domain is according to the general numbering for VH domain given by Kabat et al. ("Kabat numbering") ("Sequence of proteins of immunological interest", US Public Health Services, NIH Bethesda, MD, Publication No. 91). Numbering of amino acid residues as disclosed in the article of Riechmann and Muyldermans 2000 (J. Immunol. Methods 240: 185-195; see, e.g., Figure 2 of this publication) is applied to VHH domain of camelid animals, and correspondingly, FR1 of an immunoglobulin single variable domain comprises amino acid residues at positions 1-30, CDR1 of an immunoglobulin single variable domain comprises amino acid residues at positions 31-35, FR2 of an immunoglobulin single variable domain comprises amino acid residues at positions 36-49, CDR2 of an immunoglobulin single variable domain comprises amino acid residues at positions 50-65, FR3 of an immunoglobulin single variable domain comprises amino acid residues at positions 66-94, CDR3 of an immunoglobulin single variable domain comprises amino acid residues at positions 95-102, and FR4 of an immunoglobulin single variable domain comprises amino acid residues at positions 103-113.

Based on examples of the immunoglobulin single variable domain sequences given herein and in WO 08/020079, in WO 06/040153 and in other references cited therein for immunoglobulin single variable domains, it will be clear that the precise number of amino acid residues will also depend on the length of the particular CDRs present in an immunoglobulin single variable domain. With respect to CDRs, as is well known in the art, there are various definitions and conventions for describing CDRs in VH or VHH fragments, such as the Kabat definition (which is based on sequence variability and is the most commonly used) and the Chothia definition (which is based on the location of the structure ring region). For example, one can refer to the website http://www.bioinf.org.uk/abs/. For the purposes of the present specification and claims, even though referring to CDRs according to Kabat may be mentioned, it is most preferred to define CDRs according to Abm definition (which is based on OxfordMolecular's AbM antibody modeling software), as it is believed that Abm definition is the best compromise of Kabat and Chothia definitions. Again one can refer to the website http://www.bioinf.org.uk/abs/.

In one embodiment, FR4 comprises the C-terminal amino acid sequence VTVSS, i.e. corresponding to residues at positions 109, 110, 111, 112 and 113. The present invention also comprises ISVDs terminating at positions 109, 110, 111 or 112. In one aspect of the invention, FR4 ends with the C-terminal amino acid sequence VTVS (positions 109-112), FR4 ends with the C-terminal amino acid sequence VTV (positions 109-111), FR4 ends with the C-terminal amino acid sequence VT (positions 109-110), or FR4 ends with the C-terminal amino acid V (position 109). The C-terminal extension may be present at the C-terminal end of the last amino acid residue (most C-terminal) of FR4 of the ISVD, for example, at the C-terminal end of the last amino acid residue such as V109, T110, V111, S112 or S113 of FR4, wherein the cysteine moiety of the invention is preferably present at or located at the C-terminus of a C-terminal extension. In one embodiment, FR4 comprises the C-terminal amino acid sequence VTVSS and the C-terminal extension is a cysteine (e.g., a polypeptide of the present invention is terminated with VTVSSC). In one embodiment, FR4 comprises the C-terminal amino acid sequence VTVS and the C-terminal extension is a cysteine (e.g., a polypeptide of the present invention is terminated with VTVSC). In one embodiment, FR4 comprises the C-terminal amino acid sequence VTV and the C-terminal extension is a cysteine (e.g., a polypeptide of the present invention is terminated with VTVC). In one embodiment, FR4 comprises the C-terminal amino acid sequence VT and the C-terminal extension is a cysteine (e.g., a polypeptide of the present invention is terminated with VTC). In one embodiment, FR4 comprises the C-terminal amino acid V and the C-terminal extension is a cysteine ( e.g., a polypeptide of the present invention is terminated with VC).

In one embodiment, the present invention relates to a dimer as described herein, wherein the ISVD is a light chain variable domain sequence (VL), a heavy chain variable domain sequence (VH), a sequence derived from a conventional four-chain antibody or derived from a heavy chain antibody.

In one embodiment, the present invention relates to a dimer as described herein, wherein the ISVD is selected from the group consisting of a single domain antibody, a domain antibody, an amino acid sequence suitable for use as a single domain antibody, an amino acid sequence suitable for use as a domain antibody, a dAb, an amino acid sequence suitable for use as a dAb, a nanobody, a VHH, a humanized VHH, and a camelized VH. Preferably, the ISVD comprises 100 to 140 amino acid residues, such as 110 to 130 amino acid residues.

Compared with the prior art, the technical solutions of the present invention have the following advantages:
Firstly, the antibody of the present invention is a humanized anti-PD-L1 single variable domain antibody with high affinity. The humanized anti-PD-L1 single variable domain antibody hzF2 specifically binds to human PD-L1 protein with high affinity, with an affinity (KD) of 1.1 nM, which is comparable to the control antibody KN035. The basic properties of high affinity and good specificity provide a theoretical basis for the inhibitory effect of hzF2 on the PD-1/PD-L1 signaling pathway; and the single variable domain antibody has more flexible application pattern and is more suitable for the development of bispecific/multispecific therapeutic antibodies, as compared with a conventional monoclonal antibody.
Secondly, the antibody of the present invention has good biological activity. hzF2 can effectively bind to human PD-L1 recombinantly expressed on a cell surface, and the EC50 for binding to human PD-L1 recombinantly expressed on CHO cells (CHO-PD-L1) is 1.01nM; hzF2 can effectively block the binding of a recombinant human PD-L1 to its receptor PD-1, with IC50 of 4.3 nM; hzF2 can block PD-1/PD-L1 signaling pathway with the activity of EC50 being 5.45 nM, as detected by using Jurkat-PD1-NFAT cell and CHO-PD-L1-CD3L cell reporter gene assay; hzF2 has good *in vivo* stability and can effectively inhibit tumor growth in an immune system humanized and melanoma A375 subcutaneously xenografted model.
Thirdly, the present invention provides multiple variants based on hzF2. Some variants show better performances than the original antibody hzF2 in such performance parameters as specificity, affinity, and the like. These different single variable domain antibody variants provide more choices for PD-L1-based tumor detection, targeted therapy, drug delivery, etc., and further enrich and expand the application potential of hzF2.

### Description of Figures

Various other advantages and benefits will become apparent to those of ordinary skill in the art upon reading the following detailed description of the preferred embodiments. The drawings are for the purpose of illustrating preferred embodiments only and are not to be considered limiting of the invention. Also, the same components are denoted by the same reference symbols throughout the drawings. In the attached drawings:
Figure 1: Inhibitory effects of single variable domain antibodies on the binding of human PD-L1 to its receptor PD-1 by ELISA assay.
Figure 2: FACS analysis of the binding activity of chF2 to a cell surface antigen.
Figure 3: ELISA analysis of the binding specificity of chF2 to a recombinant PD-L1.
Figure 4: Analysis of the binding specificity of chF2 to PD-L1 on cell surface.
Figure 5: Analysis result of the inhibitory effect of hzF2 on the binding of human PD-L1 to its receptor PD-1.
Figure 6: Assessment of *in vitro* blocking activity of an anti-PD-L1 VHH antibody-Fc fusion protein by using a reporter gene system.
Figure 7: Drug-time curve of hzF2 in Balb/C mice upon single dose.
Figure 8: Experimental result (tumor volume) of antitumor efficacy of hzF2 in a murine colon cancer model using human PD-L1 transgenic mice subcutaneously allografted with MC38-hPDL1.
Figure 9: Experimental result (tumor weight) of antitumor efficacy of hzF2 in a murine colon cancer model using human PD-L1 transgenic mice subcutaneously allografted with MC38-hPDL1.

### Detailed Description of the Embodiments

Exemplary embodiments of the present disclosure will be described in more detail below with reference to the attached drawings. While exemplary embodiments of the present disclosure are shown in the drawings, it should be understood that the present disclosure can be achieved in various forms and should not be limited by the embodiments set forth herein. Rather, these embodiments are provided in order to understand the present disclosure more thoroughly, and to convey the scope of the present disclosure fully to those skilled in the art.

### Example 1. Construction of immunized camel single variable domain antibody library via phage display

An antigen was used to immunize camels. Peripheral blood mononuclear cells (PBMCs) were isolated and total RNA was extracted for reverse transcription. The product from reverse transcription was used as a template to amplify the heavy chain variable domain of the heavy-chain antibody (VHH). The heavy chain variable domain was linked into a phage display vector, and the obtained vector was electrotransfected into E. coli TG1 competent cells to construct a camel immunization library. Particularly, camels were immunized once every two weeks, and 4 times in total. The immunization was performed by injection of 0.8 mg PD-L1 extracellular region recombinant protein (in-house expression and purification, gene sequence ID number: NP_054862.1, 19aa-238aa), adjuvanted with incomplete Freund's adjuvant (Sigma, Cat.: F5506-10ml). Each injection was performed subcutaneously in a multi-point way. Two weeks after each immunization, 1 mL of blood was collected to separate the serum. The immunogen was used as the detection antigen to determine the titers of whole antibody (IgG) and of heavy chain antibody (HcAb) in serum by ELISA. When the serum titer fulfilled the requirements for constructing a library, 100 mL of camel peripheral blood was collected and PBMCs were isolated using an isolation kit (Tianjin Haoyang, Cat.: TBD2011CM), and the total RNA of PBMCs was extracted for reverse transcription to obtain cDNA. The obtained cDNA was used as a template for subsequent amplification of VHH fragments. Primers for VHH antibody library construction were designed based on the genes of camel-derived VHH antibodies retrieved from relevant literatures and databases, and synthesized. The gene sequences of the antibody variable regions were amplified by PCR. The phage display vectors and the amplified antibody fragments were then digested respectively with endonucleases, and ligated together using T4 ligase to construct ligation products. The ligation products were introduced into TG1 strain by electrotransfection technology. Finally, an immunized camel anti-human PD-L1 VHH antibody library having a concentration of 1.8×10⁸/milliliter was constructed for the screening of specific anti-human PD-L1 single variable domain antibodies. To detect the correctness of the library, 50 clones were randomly selected for colony PCR, and the results showed that the insertion percentage had reached 100%.

### Example 2. Screening of specific anti-human PD-L1 single variable domain antibodies

The constructed camel immunization library was screened by a solid-phase screening method, to obtain phage-displayed specific single variable domain antibodies.
(1) Displaying original library. The camel immunization library was inoculated to 2YT medium containing ampicillin and tetracycline, grown to logarithmic growth phase, then M13 helper phage was added thereto, followed by adding kanamycin, placed overnight at a lower temperature condition to display original library. The culture supernatant was collected the next day, and the phage was concentrated by PEG precipitation to obtain a display product having a high-titer antibody library for subsequent screening.
(2) Screening. The specific antibodies were screened by a solid-phase method. The specific antigen was coated on the surface of an immune tube. The immune tube and the antibody library were blocked respectively by a blocking agent, then the antibody library was added to the immune tube, and incubated, then washed repeatedly, and finally an acid having pH2.2 was used for elution. The eluate was neutralized to neutrality, then incubated with XL-Blue in the logarithmic growth phase for infection, and subjected to further phage display. Specific phage particles were recovered. After screening 2-3 rounds, monoclones were to be identified.
(3) Identification. XL-Blue bacteria infected by the recovered specific phage particles were inoculated on plates, and individual clones were identified after growing into colonies. Individual clones were picked out and cultured to logarithmic growth phase, then M13 helper phage was added to infect, followed by adding kanamycin; then placed overnight at 30°C. The culture supernatant was collected the next day and added to an enzyme-linked plate coated with PD-L1 to perform ELISA reaction. Phagemids (phage display vectors comprising antibody genes) were extracted from the reaction positive clones, and sequenced to determine the VHH antibody gene sequences. Five phage-displayed single variable domain antibodies (VHHs), i.e. 1-4G1, 1-6C4, 2-3D6, 2-5B7, 2-2F2, capable of binding to the human PD-L1 recombinant protein were obtained through screening.

### Example 3. Preliminary identification of specific anti-human PD-L1 single variable domain antibodies

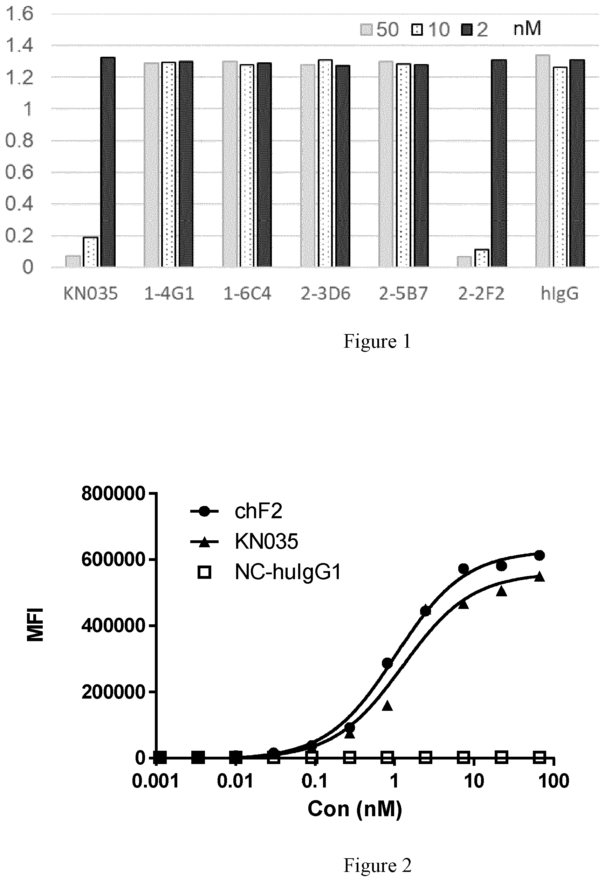

The obtained five single-domain VHH antibodies were expressed by inducing E. coli TG1, and the induction conditions were 1 mM IPTG, at 30°C, and 150 rpm, overnight culture. Bacterial samples upon induced expression were disrupted by sonication and filtered, then purified using a nickel column via affinity, and ultrafiltered, to obtain single-domain VHH antibodies. The inhibitory effect of the single domain VHH antibodies on the binding of human PD-L1 to its receptor PD-1 was then tested by ELISA. Particularly, a fusion protein of human PD-1 extracellular region and human Fc (PD-1-hFc, PD-1 sequence ID number: NP_005009.2, 21aa-167aa) was coated on an ELISA plate at a concentration of 0.5 µg/mL. Then the plate was placed at 4°C overnight, and blocked with 5% BSA for 60 min at 37°C in a thermostatic incubator. The single-domain VHH antibodies (at concentrations of 50, 10, 2nM) and 1 µg/mL PD-L1-mFc were co-incubated and reacted in a thermostatic incubator at 37 °C for 60 min. The plate was washed 4 times with PBST; then added 1:5000 diluted HRP-anti-mouse Fc (Jackson Immuno Research, Cat.: 115-035-071), reacted for 45 min, and added TMB (Beijing Taitianhe Biology, Cat.: ME142) substrate to develop color for 15 min. After adding 2M HCl to stop the reaction, the plate was read and recorded about the absorbance value of A450nm-630nm of the well plate by a reader taking 630 nm as the reference wavelength, and 450 nm as the detection wavelength. The results showed that 2-2F2 could effectively block the binding of recombinant human PD-L1 to its receptor PD-1, illustrating that 2-2F2 had good blocking activity (Figure 1). This molecule, abbreviated as VHH-F2, was selected as the original molecule for subsequent development. The amino acid sequence of the variable region of the single variable domain antibody was shown as SEQ ID NO.1, and the nucleotide sequence of the variable region was shown as SEQ ID NO.2.

Specific primers were designed, a positive cloned phagemid was used as the template, and the variable region gene of the camel-derived antibody VHH-F2 was obtained by PCR. Then, the variable region gene was cloned into a eukaryotic expression vector comprising human Fc (IgG1, hFc) encoding gene by enzymatic digestion and ligation. After obtaining an expression plasmid with the correct sequence, it was transfected into 293F cells for transient expression, then the expression product was purified by Protein A, and finally a fusion protein of a human-camel chimeric single variable domain antibody (VHH-F2-human-Fc chimeric antibody, abbreviated as "chF2") was obtained. The full-length amino acid sequence of the chF2 antibody molecule was shown as SEQ ID NO.3, and the nucleotide sequence was shown as SEQ ID NO.4.

With reference to the Envafolimab antibody sequence published by WHO (WHO Drug Information, Vol. 33, No. 3, 2019, Page634-635, Envafolimab), the KN035 variable region gene was completely synthesized, and the amino acid sequence of the KN035 variable region was shown as SEQ ID NO.5, and the nucleotide sequence was shown as SEQ ID NO.6. The same strategy as above for constructing chF2 was used to obtain a fusion protein having KN035 variable region and Fc, and abbreviated as KN035.

### Example 4. Binding activity analysis of the anti-human PD-L1 chimeric single variable domain antibody

### Method 1. BLI assay for binding activity

Using the Octet QKe system instrument from Fortebio Company, the ability of chF2 to bind corresponding recombinant antigen was determined by using the capture antibody (AHC) biological probe against the Fc fragment of the human antibody to capture the Fc fragment of the antibody. During the measurement, chF2 was diluted with PBS buffer to 4 µg/mL, and flowed over the surface of AHC probe (Cat.: 18-0015, PALL) for 120 s. A human PD-L1 recombinant protein was used as the mobile phase to interact with the antibody captured on the chip surface. The recombinant PD-L1 protein concentration was 60 nM. The binding time for each antigen was 300s, and the final dissociation time was 300s. The results (Table 1) showed that under the present experimental conditions, the chF2 bound to the recombinant PD-L1 protein in higher affinity, which was comparable to that of the control antibody KN035.

**Table 1. Measurement of affinity of chF2 and KN035 to the human PD-L1 recombinant protein**

| | **KD value(M)** | **kon(1/Ms)** | **kdis(1/s)** |
|---|---|---|---|
| chF2 | 1.11E-09 | 2.24E+05 | 2.48E-04 |
| KN035 | 1.50E-09 | 2.33E+05 | 3.50E-04 |

### Method 2. Binding activity analysis via FACS

Cells (CHO-PD-L1-CD3L cells) were collected after centrifugation, divided into 5×10⁵ cells/sample/100 µL. Gradient diluted single variable domain antibody was added to the cells at a final concentration of 66nM as the highest concentration, 3-fold serial dilution to make 10 gradients; then incubated on ice for 2h. The cells were washed twice with ice-cold PBS (containing 0.05% Tween). FITC-labeled anti-human Fc secondary antibody (Cat.: F9512, Sigma) was added, and incubated on ice for 1h. The cells were washed twice with ice-cold PBS (containing 0.05% Tween), resuspended in 200 µL of Flow Cytometry Buffer, and the mean fluorescence intensity (MFI) of the cells was detected by flow cytometer (Model B49007AD, SNAW31211, BECKMAN COULTER). The detection results showed that chF2 and KN035 had comparable binding activities to PD-L1 expressed on the cell surface, with half effective binding concentration (EC50) values of 1.04nM and 1.27nM, respectively (Figure 2).

### Example 5. Analysis of the specificity of the anti-human PD-L1 chimeric single variable domain antibody

### Method 1. Identification of the specificity of the chimeric antibody to the recombinant antigen by ELISA

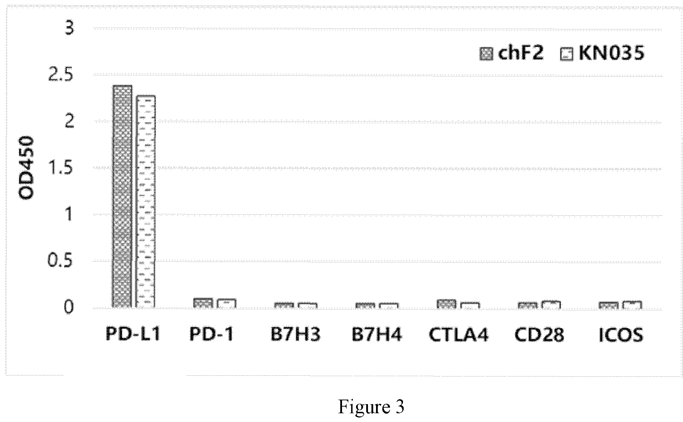

Recombinant human proteins (PD-L1, PD-1, B7H3, B7H4, CTLA4, CD28, ICOS, etc.) were diluted with PBS to 1 µg/mL, then used 100 µL/well to coat enzyme-linked plate, overnight at 4°C. The plate was blocked by 5% BSA blocking solution in a thermostatic incubator at 37°C for 60 min, then washed 3 times with PBST; added chF2 diluted to 1 µg/mL, reacted at 37°C for 60 min, and washed 4 times with PBST; added HRP-Anti-human IgG diluted 1:5000 for 45 min reaction, and washed 4 times with PBST; finally, added TMB substrate for color development. The reaction was performed in a thermostatic incubator at 37°C for 15 min, and stopped with 2M HCl. The plate was read and recorded about the absorbance value of A450nm-630nm of the well plate by a reader taking 630 nm as the reference wavelength, and 450 nm as the detection wavelength. The results (Fig. 3) showed that chF2 specifically bound to PD-L1, but not to other recombinant proteins.

### Method 2. BLI assay for identifying species specificity

The Octet QKe system instrument from Fortebio Company was used to determine whether chF2 could bind to recombinant monkey PD-L1 and recombinant murine PD-L1 by using the capture antibody (AHC) biological probe of anti-human antibody Fc segment to capture the Fc segment of the antibody. During the measurement, chF2 was diluted to 4 µg/mL with PBS buffer and flowed over the surface of AHC probe (PALL, Cat.: 18-0015) for 120 s. The monkey PD-L1 recombinant protein and the murine PD-L1 recombinant protein were used as mobile phases to interact with the antibodies captured on the surface of the chip. The PD-L1 recombinant protein concentration was 60 nM. The binding time of each antigen was 300s, and the final dissociation time was 300s. The results showed that both chF2 and KN035 bound to recombinant monkey PD-L1 protein with comparable affinity, but not to recombinant murine PD-L1 protein (Table 2).

**Table 2. Analysis of the binding activity of chF2 and KN035 to monkey and murine PD-L1 recombinant proteins**

| | | **KD value(M)** | **kon(1/Ms)** | **kdis(1/s)** |
|---|---|---|---|---|
| hzF2 | Monkey PD-L1 | 1.40E-09 | 2.18E+05 | 3.05E-04 |
| | Murine PD-L1 | No binding signal | | |
| KN035 | Monkey PD-L1 | 1.32E-09 | 2.13E+05 | 2.81E-04 |
| | Murine PD-L1 | No binding signal | | |

### Method 3. Identification of chimeric antibody specificity by FACS

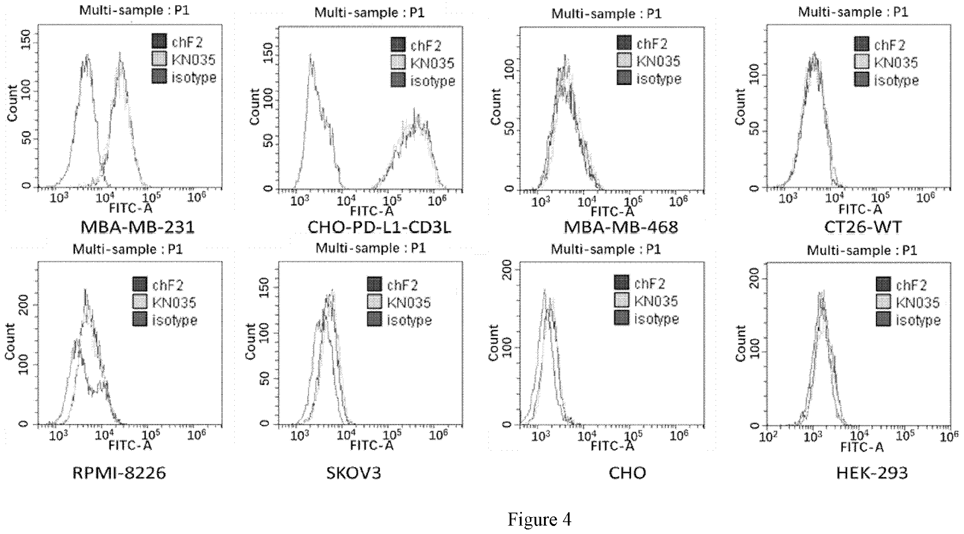

Cells were collected after centrifugation, divided into 3×10⁵ cells/sample/100 µL, and 20 µg/ml of the single variable domain antibody was added to the cells. The cells were incubated on ice for 2h, and washed twice with ice-cold PBS (containing 0.05% Tween). FITC-labeled anti-human Fc secondary antibody (Sigma, Cat.: F9512) was added, and incubated on ice for 1h. The cells were washed twice with ice-cold PBS (containing 0.05% Tween), resuspended in 200 µL of Flow Cytometry Buffer, and detected by a flow cytometer. The detection results (Figure 4) showed that the reactivity of chF2 to each of the tumor cell types was exactly the same as that of the control antibody KN035, and both of them specifically bound to cell lines expressing human PD-L1, but not to non-PD-L1 expressing cell lines.

### Example 6. Humanization of chF2

A human heavy chain variable region having the highest homology to the variable region of the camel-derived antibody VHH-F2 was select for the framework in the human heavy chain variable region. The variable region of VHH-F2 was humanized through CDR grafting and retaining partial amino acids for providing the support structure. A fusion protein (VHH-F2-human-Fc humanized antibody, abbreviated as hzF2) of a humanized single variable domain antibody was designed, having a variable region amino acid sequence shown as SEQ ID NO.7, and full-length amino acid sequence shown as SEQ ID NO.9. The nucleotide sequence of hzF2 variable region shown as SEQ ID NO.8 was synthesized, and a recombinant expression vector of hzF2 was constructed. The constructed full-length hzF2 variable region nucleotide sequence was shown as SEQ ID NO.10. After eukaryotic expression, the affinity of hzF2 was determined by BLI method, and correlation analysis was performed.

The antibody clone hzF2 was further mutated to obtain a large number of variant antibodies. The amino acid sequences of the CDR regions of the variants were shown in Table 3, and the amino acid sequences of the variable regions of the variants were shown in Table 4 (SEQ ID NO.11∼SEQ ID NO. 26), and the nucleotide sequence of the variable regions of the variants were shown as SEQ ID NO.27∼SEQ ID NO.42, and the changes in the binding constant and dissociation constant of some variants were shown in Table 5.

**Table 3. CDR region amino acid sequences and affinity changes of hzF2 variants**

| Variant | Mutated sequence | | | Affinity KD |
|---|---|---|---|---|
| | CDR1 | CDR2 | CDR3 | |
| hzF2 | RDSDDGASCMG | IIFNAGERTDYGDSVKG | VWCGSWVARS | 1.47nM |
| hzF2-m1 | RDSDDGASSMG | IIFNAGERTDYGDSVKG | VWSGSWVARS | 2.80nM |
| hzF2-m2 | GDSDDGASCMG | IIFNAGERTDYGDSVKG | VWCGSWVARS | 1.26nM |
| hzF2-m3 | RDSSSGASCMG | IIFNAGERTDYGDSVKG | VWCGSWVARS | 7.80nM |
| hzF2-m4 | RDSNDGASCMG | IIFNAGERTDYGDSVKG | VWCGSWVARS | 9.36nM |
| hzF2-m5 | RDSSSAASCMG | IIFNAGERTDYGDSVKG | VWCGSWVARS | 5.05nM |
| hzF2-m6 | RDSDEGASCMG | IIFQAGERTDYGDSVKG | VWCGSWVARS | 1.10nM |
| hzF2-m7 | RDSDDSASCMG | IIFNAGERTDYGDSVKG | VWCGSWVARS | 4.59nM |
| hzF2-m8 | RDSDDGASCMG | IIFNVGERTDYGDSVKG | VWCGSWVARS | 1.18nM |
| hzF2-m9 | RDSDEGASCMG | IIFNAGERTDYGDSVKG | VWCGSWVARS | 1.34nM |
| hzF2-m 10 | RDSDDGASCMG | IIFNAGERTDYGDSVKG | VYCGSWVARS | 4.31nM |
| hzF2-m 11 | RDSDDGASCMG | IIFNAGERTDYGDSVKG | VYCGSYVARS | 3.24nM |
| hzF2-m12 | RDSDEGASCMG | IIFNVGERTDYGDSVKG | VWCGSWVARS | 1.54nM |
| hzF2-m13 | RDSDDGASCMG | IIFNAGERTDYGDSVKG | VFCGSFVARS | 4.26nM |
| hzF2-m14 | RDSDEGASCMG | IIFNVGERTDYGDSVKG | VYCGSYVARS | 3.81nM |
| hzF2-m15 | RDSDEGASCMG | IIFNVGERTDYGDSVKG | VFCGSYVARS | 4.75nM |
| hzF2-m16 | RDSDEGASCMG | IIFNVGERTDYGDSVKG | VFCGSFVARS | 3.18nM |

**Table 4. Variable region amino acid sequences of hzF2 variants**

| Variant name | Variant amino acid sequence |
|---|---|
| hzF2-m1 SEQ ID NO.11 | EVQLVESGGGLVQPGGSLRLSCAASRDSDDGASSMGWFRQAPGKGLE GVAIIFNAGERTDYGDSVKGRFTISRDNAKNTLYLQMNSLRAEDTAV YYCATVWSGSWVARSWGQGTLVTVSS |
| hzF2-m2 SEQ ID NO. 12 | EVQLVESGGGLVQPGGSLRLSCAASGDSDDGASCMGWFRQAPGKGL EGVAIIFNAGERTDYGDSVKGRFTISRDNAKNTLYLQMNSLRAEDTAV YYCATVWCGSWVARSWGQGTLVTVSS |
| hzF2-m3 SEQ ID NO. 13 | EVQLVESGGGLVQPGGSLRLSCAASRDSSSGASCMGWFRQAPGKGLE GVAIIFNAGERTDYGDSVKGRFTISRDNAKNTLYLQMNSLRAEDTAV YYCATVWCGSWVARSWGQGTLVTVSS |
| hzF2-m4 SEQ ID NO. 14 | EVQLVESGGGLVQPGGSLRLSCAASRDSNDGASCMGWFRQAPGKGL EGVAIIFNAGERTDYGDSVKGRFTISRDNAKNTLYLQMNSLRAEDTAV YYCATVWCGSWVARSWGQGTLVTVSS |
| hzF2-m5 SEQ ID NO. 15 | EVQLVESGGGLVQPGGSLRLSCAASRDSDDAASCMGWFRQAPGKGL EGVAIIFNAGERTDYGDSVKGRFTISRDNAKNTLYLQMNSLRAEDTAV YYCATVWCGSWVARSWGQGTLVTVSS |
| hzF2-m6 SEQ ID NO. 16 | EVQLVESGGGLVQPGGSLRLSCAASRDSDEGASCMGWFRQAPGKGLE GVAIIFNAGERTDYGDSVKGRFTISRDNAKNTLYLQMNSLRAEDTAV YYCATVWCGSWVARSWGQGTLVTVSS |
| hzF2-m7 SEQ ID NO. 17 | EVQLVESGGGLVQPGGSLRLSCAASRDSDDSASCMGWFRQAPGKGLE GVAIIFNAGERTDYGDSVKGRFTISRDNAKNTLYLQMNSLRAEDTAV YYCATVWCGSWVARSWGQGTLVTVSS |
| hzF2-m8 SEQ ID NO. 18 | EVQLVESGGGLVQPGGSLRLSCAASRDSDDGASCMGWFRQAPGKER EGVAIIFNVGERTDYGDSVKGRFTISRDNAKNTLYLQMNSLRAEDTAV YYCATVWCGSWVARSWGQGTLVTVSS |
| hzF2-m9 SEQ ID NO. 19 | EVQLVESGGGLVQPGGSLRLSCAASRDSDEGASCMGWFRQAPGKERE GVAIIFNAGERTDYGDSVKGRFTISRDNAKNTLYLQMNSLRAEDTAVY YCATVWCGSWVARSWGQGTLVTVSS |
| hzF2-m10 SEQ ID | EVQLVESGGGLVQPGGSLRLSCAARDSDDGASCMGWFRQAPGKERE GVIIFNAGERTDYGDSVKGRFTISRDNAKNTLYLQMNSLRAEDTAVYY |
| NO.20 | CATVYCGSWVARSWGQTLVTVSS |
| hzF2-m 11 SEQ ID NO.21 | EVQLVESGGGLVQPGGSLRLSCAARDSDDGASCMGWFRQAPGKERE GVIIFNAGERTDYGDSVKGRFTISRDNAKNTLYLQMNSLRAEDTAVYY CATVYCGSYVARSWGQGTLVTVSS |
| hzF2-m12 SEQ ID NO.22 | EVQLVESGGGLVQPGGSLRLSCAARDSDEGASCMGWFRQAPGKEREG VIIFNVGERTDYGDSVKGRFTISRDNAKNTLYLQMNSLRAEDTAVYYC ATVWCGSWVARSWGQGTLVTVSS |
| hzF2-m13 SEQ ID NO.23 | EVQLVESGGGLVQPGGSLRLSCAARDSDDGASCMGWFRQAPGKERE GVIIFNAGERTDYGDSVKGRFTISRDNAKNTLYLQMNSLRAEDTAVYY CATVFCGSFVARSWGQGTLVTVSS |
| hzF2-m14 SEQ ID NO.24 | EVQLVESGGGLVQPGGSLRLSCAARDSDEGASCMGWFRQAPGKEREG VIIFNVGERTDYGDSVKGRFTISRDNAKNTLYLQMNSLRAEDTAVYYC ATVYCGSYVARSWGQGTLVTVSS |
| hzF2-m15 SEQ ID NO.25 | EVQLVESGGGLVQPGGSLRLSCAARDSDEGASCMGWFRQAPGKEREG VIIFNVGERTDYGDSVKGRFTISRDNAKNTLYLQMNSLRAEDTAVYYC ATVFCGSYVARSWGQGTLVTVSS |
| hzF2-m16 SEQ ID NO.26 | EVQLVESGGGLVQPGGSLRLSCAARDSDEGASCMGWFRQAPGKEREG VIIFNVGERTDYGDSVKGRFTISRDNAKNTLYLQMNSLRAEDTAVYYC ATVFCGSFVARSWGQGTLVTVSS |

**Table 5. Detection results of the affinities of hzF2 variants to a human PD-L1 recombinant protein**

| | **KD value(M)** | **kon(1/Ms)** | **kdis(1/s)** |
|---|---|---|---|
| HzF2 | 1.40E-09 | 2.18E+05 | 3.05E-04 |
| hzF2-m1 | 2.80E-09 | 6.79E+05 | 1.90E-03 |
| hzF2-m2 | 1.26E-09 | 5.62E+05 | 7.09E-04 |
| hzF2-m3 | 7.80E-09 | 6.24E+05 | 4.87E-03 |
| hzF2-m4 | 9.36E-09 | 6.95E+05 | 6.50E-03 |
| hzF2-m5 | 5.05E-09 | 3.36E+05 | 1.70E-03 |
| hzF2-m6 | 1.10E-09 | 3.15E+05 | 3.45E-04 |
| hzF2-m7 | 4.59E-09 | 3.41E+05 | 1.57E-03 |
| hzF2-m8 | 1.18E-09 | 3.56E+05 | 4.19E-04 |
| hzF2-m9 | 1.34E-09 | 3.24E+05 | 4.34E-04 |

### Example 7. Inhibitory effect of hzF2 on the binding of human PD-L1 to its receptor PD-1 by ELISA assay

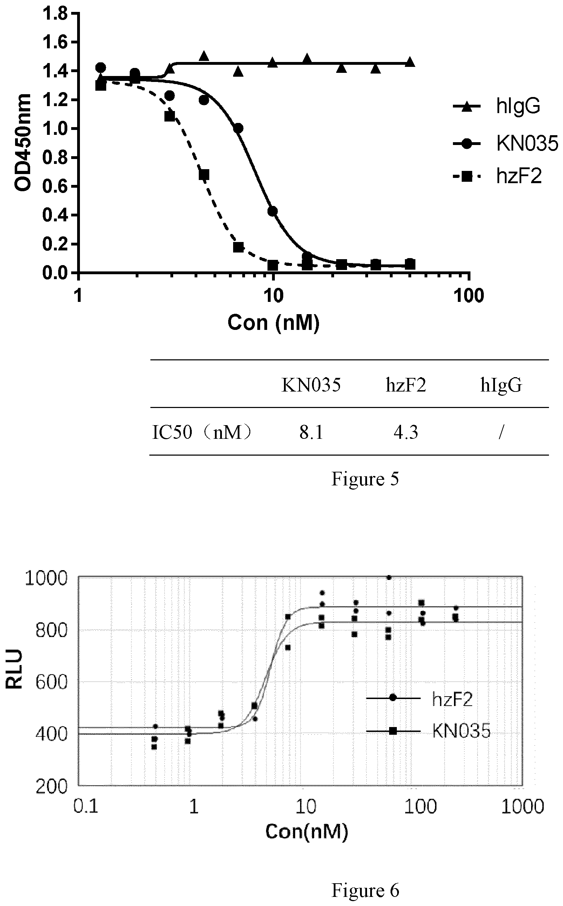

Human PD-1-hFc (PD-1 Sequence No. NP_005009.2, 21aa-167aa) was diluted with PBS to 0.5 µg/mL, coated on an ELISA plate overnight at 4°C. Then the plate was blocked with 5% BSA for 60 min at 37°C in a thermostatic incubator. Gradient dilutions of hzF2 and control antibody KN035, and an isotype control NC-hIgG1 (initial working concentration of 50 nM, 1.5-fold dilution to 10 concentration gradients), were added, then PD-L1-mFc (PD-L1 Sequence No. NP_054862.1, 19aa-238aa) was added at a working concentration of 0.5 µg/mL, co-incubated and reacted in a thermostatic incubator at 37 °C for 60 min. The plate was washed 4 times with PBST; then added 1:5000 diluted HRP-anti-mouse Fc (Cat.: 115-035-071, Jackson Immuno Research) thereto, reacted for 45 min, and added TMB (Beijing Taitianhe Biology, Cat.: ME142) substrate to develop color for 15 min. After adding 2M HCl to stop the reaction, the plate was read and recorded about the absorbance value of A450nm-630nm of the well plate by a reader taking 630 nm as the reference wavelength, and 450 nm as the detection wavelength. The results showed (Figure 5) that hzF2 could effectively block the binding of recombinant human PD-L1 to its receptor PD-1, with a half effective inhibitory concentration (IC50) of 4.3nM.

### Example 8. Evaluation of cell blocking activity by PD-1 and PD-L1 reporter gene method

The blocking effect of hzF2 on the PD-1 and PD-L1 pathway was assayed by the Reporter Gene Assay (RGA) using Jurkat-PD1-NFAT cells and CHO-PD-L1-CD3L cells. The details were as follows: CHO-PD-L1-CD3L cells in the logarithmic growth phase were adjusted to a cell density of 5×10⁵ cells/ml, plated at 100 µl/well, and placed overnight. Antibody samples were stepwise pre-diluted to 20 µg/ml with culture medium, and then diluted in 2-fold gradient for a total of 10 points. The diluted samples were added at 50 µl/well to the cells cultured overnight. At the same time, Jurkat-PD1-NFAT cells at a concentration of 2×10⁶ cells/ml were added at 50 µl/well. The plate was incubated in a cell incubator for 6 h. An appropriate amount of Bio-Gio^{™} Luciferase substrate was taken out 1~2 hours in advance and thawed, then placed at room temperature in the dark. The cell plate was taken out from the incubator, equilibrated to room temperature (about 10-15 min), added Bio-Gio^{™} Luciferase substrate at 100 µl/well. The cell plate was placed in a microplate thermostatic shaker, and incubated in the dark at 800 rpm for 20 min. The multi-function microplate reader was set to Luminescence mode, selecting 500 for Integration (the default value of the instrument), and reading the RLU. SoftMax software was used to analyze the data, taking the sample concentrations as the X-axis and the RLU average detection values as the Y-axis, and selecting a four-parameter equation to draw a standard curve. According to the EC₅₀ value of the curve fitting results of the reference sample and the test sample, the relative biological activity of the test sample was calculated. The results were shown in Figure 6. The blocking activity of hzF2 on PD-L1 and PD-1 was substantially comparable to that of KN035, with EC50 of hzF2 being 5.45nM; and EC50 of KN035 being 4.90nM, respectively.

### Example 9: Determination of the half-life of hzF2 in mice

Healthy female 5-week-old nude mice, grouped into three mice per group, were injected with the antibody through the tail vein at a single dose of 15 mg/kg. 2h, 4h, 8h, 24h, 48h, 96h, 144h, 196h after the administration, tail vein blood samples were collected respectively, centrifuged to separate serum, and stored at -20°C, for study the pharmacokinetic properties of the antibody. After the collection of all blood samples was completed, the following procedure was performed. A 96-well enzyme-linked plate was coated by PD-L1-His (Sequence No. NP_054862.1, 19aa-238aa) at 0.5µg/ml, 100µl/well, and placed overnight at 4°C, then washed 3 times with PBS. The plate was added 5% BSAPBS, and blocked at 37°C for 60 min, washed 3 times with PBST; then added serum samples to be tested (10,000-fold dilution, 20,000-fold dilution) and standard to set hzF2 standard curve wells (initial concentration of 0.05µg/mL, 2-fold serial dilution, 12 dilution gradients), incubated at 37°C for 60 min, washed 4 times with PBST; added 1:5000 diluted HRP-goat anti-human IgG (Fcr) (Cat.: 109-035-098, Jackson Immuno Research), incubated at 37°C for 40 min, washed 4 times with PBST; added TMB substrate (Cat.: ME142, Beijing Taitianhe Biotechnology Co., Ltd.) for color development. After incubating at 37°C for 10 min, the plate was added 2M HCl to stop the reaction, then read and recorded about the absorbance value of A450nm-630nm of the well plate by a reader taking 630 nm as the reference wavelength, and 450 nm as the detection wavelength. Time-antibody concentration curve was plotted, taking the concentrations of the standard antibody as the Y-axis and the OD values as the X-axis, and linear fitting was performed. The drug half-life T_{1/2} was T_{1/2}=|0.693/k|, which was calculated according to the equation.

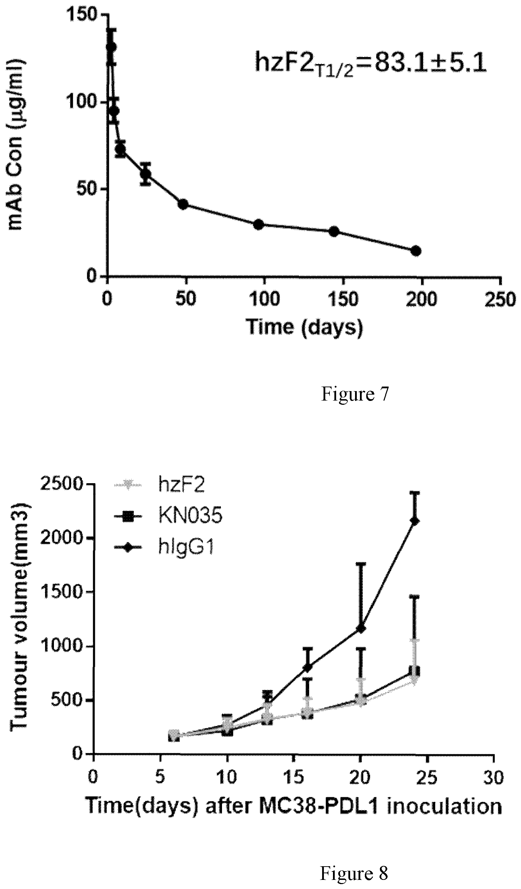

The final results (Figure 7) showed that under the present conditions, the in vivo half-life of hzF2 in mice was 83.1 hours, suggesting that hzF2 had good in-vivo half-life and stability.

### Example 10: Detection of antitumor efficacy of hzF2 in a murine colon cancer model using human PD-L1 transgenic mice subcutaneously allografted with MC38-hPDL1

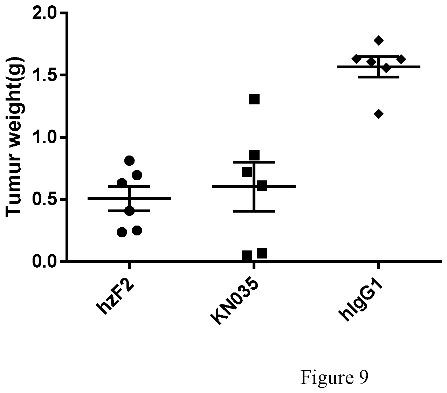

The murine colon cancer cell line MC38-hPDL1 with high expression of human PDL1 was inoculated to the subcutaneous site of the right anterior rib of female B6-hPDL1 mice (PD-L1 humanized mice with gene background of C57). When the tumor grew to about 100 mm³, the mice were grouped into hzF2, KN035 or isotype control IgG groups, and administered said drugs at a dose of 10 mg/kg, twice a week, totally administered 6 times. The tumor volume and body weight were measured upon each administration, and the relationships between the changes of the body weight and the tumor volume of the tumor-bearing mice and the administration time were recorded. At the end of the experiment, the tumor-bearing mice were euthanized, and the tumors were removed, weighed, and photographed. The tumor volume ratio (T/C) and the tumor growth inhibition rate (1-T/C) of the treatment group relative to the control group were calculated and statistically analyzed. The results showed that the test drug hzF2 effectively inhibited tumor growth (Figure 8, Figure 9).

**The amino acid and nucleotide sequences relevant to the application are as follows:**
SEQ ID NO.1: Variable region amino acid sequence of the camel-derived single variable domain antibody VHH-F2 
QVQLQESGGGSVQTGGSLRLACAVSRDSDDGASCMGWFRQAPGKGREGVAIIFNAGE
RTDYGDSVKGRFTISQDNAKNTLFLQMNSLKPEDSAMYYCATVWCGSWVARSFGQGTQV
TVSS
 wherein
   heavy chain CDR1 amino acid (SEQ ID NO.43): RDSDDGASCMG
   heavy chain CDR2 amino acid (SEQ ID NO.44): IIFNAGERTDYGDSVKG
   heavy chain CDR3 amino acid (SEQ ID NO.45): VWCGSWVARS
SEQ ID NO.2: Variable region nucleotide sequence of the camel-derived single variable domain antibody VHH-F2 
CAGGTGCAGCTGCAGGAGTCTGGAGGAGGCTCGGTGCAGACTGGAGGGTCTCTGA
GACTCGCCTGTGCAGTCTCTAGAGACAGTGACGACGGTGCCAGCTGTATGGGGTGGTTC
CGCCAGGCTCCAGGGAAGGGGCGCGAGGGGGTCGCAATCATTTTTAATGCTGGTGAACG
TACCGACTATGGCGACTCCGTGAAGGGCCGATTCACCATCTCCCAAGACAACGCCAAGA
ACACGCTGTTTCTACAAATGAACAGCCTGAAACCTGAGGACAGTGCCATGTACTATTGT
GCGACAGTTTGGTGTGGTTCTTGGGTCGCGCGTTCTTTCGGCCAGGGGACCCAGGTCAC
CGTCTCCTCA
 wherein:
   heavy chain CDR1 nucleotide:
      AGAGACAGTGACGACGGTGCCAGCTGTATGGGG
   heavy chain CDR2 nucleotide:
      ATCATTTTTAATGCTGGTGAACGTACCGACTATGGCGACTCCGTGAAGGGC
   heavy chain CDR3 nucleotide:
      GTTTGGTGTGGTTCTTGGGTCGCGCGTTCT
SEQ ID NO.3: Full-length amino acid sequence of the chimeric single variable domain antibody chF2 QVQLQESGGGSVQTGGSLRLACAVSRDSDDGASCMGWFRQAPGKGREGVAIIFNAGE 
RTDYGDSVKGRFTISQDNAKNTLFLQMNSLKPEDSAMYYCATVWCGSWVARSFGQGTQV
TVSSASEPKSSDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPG
 wherein:
   heavy chain CDR1 amino acid: RDSDDGASCMG
   heavy chain CDR2 amino acid: IIFNAGERTDYGDSVKG
   heavy chain CDR3 amino acid: VWCGSWVARS
SEQ ID NO.4: Full-length nucleotide sequence of the chimeric single variable domain antibody chF2 
CAGGTGCAGCTGCAGGAGTCTGGAGGAGGCTCGGTGCAGACTGGAGGGTCTCTGA
GACTCGCCTGTGCAGTCTCTAGAGACAGTGACGACGGTGCCAGCTGTATGGGGTGGTTC
CGCCAGGCTCCAGGGAAGGGGCGCGAGGGGGTCGCAATCATTTTTAATGCTGGTGAACG
TACCGACTATGGCGACTCCGTGAAGGGCCGATTCACCATCTCCCAAGACAACGCCAAGA
ACACGCTGTTTCTACAAATGAACAGCCTGAAACCTGAGGACAGTGCCATGTACTATTGT
GCGACAGTTTGGTGTGGTTCTTGGGTCGCGCGTTCTTTCGGCCAGGGGACCCAGGTCAC
CGTCTCCTCAGCTAGCGAGCCCAAATCTAGCGACAAAACTCACACATGCCCACCGTGCCCAG
CACCTGAACTCCTGGGGGGACCGTCAGTCTTCCTCTTCCCCCCAAAACCCAAGGACACCCTC
ATGATCTCCCGGACCCCTGAGGTCACATGCGTGGTGGTGGACGTGAGCCACGAAGACCCTG
AGGTCAAGTTCAACTGGTACGTGGACGGCGTGGAGGTGCATAATGCCAAGACAAAGCCGCG
GGAGGAGCAGTACAACAGCACGTACCGTGTGGTCAGCGTCCTCACCGTCCTGCACCAGGAC
TGGCTGAATGGCAAGGAGTACAAGTGCAAGGTCTCCAACAAAGCCCTCCCAGCCCCCATCGA
GAAAACCATCTCCAAAGCCAAAGGGCAGCCCCGAGAACCACAGGTGTACACCCTGCCTCCAT
CTCGGGATGAGCTGACCAAGAACCAGGTCAGCCTGACCTGCCTGGTCAAAGGCTTCTATCCC
AGCGACATCGCCGTGGAGTGGGAGAGCAATGGGCAGCCGGAGAACAACTACAAGACCACGC
CTCCCGTGCTGGACTCCGACGGCTCCTTCTTCCTCTATAGCAAGCTCACCGTGGACAAGAGC
AGGTGGCAGCAGGGGAACGTCTTCTCATGCTCCGTGATGCATGAGGCTCTGCACAACCACTA
CACGCAGAAGAGCCTCTCCCTGTCCCCGGGT
 wherein:
   heavy chain CDR1 nucleotide:
      AGAGACAGTGACGACGGTGCCAGCTGTATGGGG
   heavy chain CDR2 nucleotide:
      ATCATTTTTAATGCTGGTGAACGTACCGACTATGGCGACTCCGTGAAGGGC
   heavy chain CDR3 nucleotide:
      GTTTGGTGTGGTTCTTGGGTCGCGCGTTCT
SEQ ID NO.5: Amino acid sequence of KN035 variable region 
QVQLVESGGGLVQPGGSLRLSCAASGKMSSRRCMAWFRQAPGKERERVAKLLTTSGS
TYLADSVKGRFTISRDNSKNTVYLQMNSLRAEDTAVYYCAADSFEDPTCTLVTSSGAFQYW
GQGTLVTVSS
 wherein:
   heavy chain CDR1 amino acid: RRCMA
   heavy chain CDR2 amino acid: KLLTTSGSTYLADSVKG
   heavy chain CDR3 amino acid: DSFEDPTCTLVTSSGAFQY
SEQ ID NO.6: Nucleotide sequence of KN035 variable region 
CAGGTGCAGCTGGTGGAGTCTGGAGGAGGCCTGGTGCAGCCTGGAGGCTCCCTGA
GGCTGTCCTGCGCTGCCTCTGGCAAGATGTCCTCCAGACGGTGCATGGCCTGGTTCCGA
CAGGCTCCTGGCAAGGAGCGAGAGCGGGTGGCCAAGCTGCTGACCACCTCCGGCTCCA
CCTACCTGGCCGACTCCGTGAAGGGACGGTTCACCATCTCCAGGGACAACTCCAAGAA
CACCGTGTACCTGCAGATGAACTCCCTGCGAGCTGAGGACACCGCCGTGTACTACTGCG
CTGCAGACTCCTTCGAGGACCCCACCTGCACCCTGGTGACCTCCTCTGGAGCCTTCCAG
TACTGGGGACAGGGCACCCTGGTGACCGTGTCCTCC
wherein:
   heavy chain CDR1 nucleotide:
      AGACGGTGCATGGCC
   heavy chain CDR2 nucleotide:
      AAGCTGCTGACCACCTCCGGCTCCACCTACCTGGCCGACTCCGTGAAGGGA
   heavy chain CDR3 nucleotide: 
GACTCCTTCGAGGACCCCACCTGCACCCTGGTGACCTCCTCTGGAGCCTTCCAGTA
C
SEQ ID NO.7: Variable region amino acid sequence of the humanized single variable domain antibody hzF2 
EVQLVESGGGLVQPGGSLRLSCAASRDSDDGASCMGWFRQAPGKGLEGVAIIFNAGER
TDYGDSVKGRFTISRDNAKNTLYLQMNSLRAEDTAVYYCATVWCGSWVARSWGQGTLVT
VSS
 wherein:
   heavy chain CDR1 amino acid: RDSDDGASCMG
   heavy chain CDR2 amino acid: IIFNAGERTDYGDSVKG
   heavy chain CDR3 amino acid: VWCGSWVARS
SEQ ID NO. 8: Variable region nucleotide sequence of the humanized single variable domain antibody hzF2 
GAGGTGCAGCTGGTGGAGTCTGGAGGTGGCCTGGTGCAGCCTGGAGGCTCCCTGA
GGCTGTCCTGCGCTGCCTCTCGGGACTCCGACGACGGAGCCTCCTGCATGGGCTGGTTC
AGGCAGGCTCCTGGCAAGGGCCTGGAGGGAGTGGCCATCATCTTCAACGCTGGCGAGC
GGACCGACTACGGCGACTCCGTGAAGGGACGGTTCACCATCTCCAGGGACAACGCCAA
GAACACCCTGTACCTGCAGATGAACTCCCTGAGAGCCGAGGACACAGCCGTGTACTACT
GCGCTACCGTGTGGTGTGGCTCCTGGGTGGCTCGGTCCTGGGGACAGGGCACCCTGGTG
ACCGTGTCCTCC
 wherein:
   heavy chain CDR1 nucleotide:
      CGGGACTCCGACGACGGAGCCTCCTGCATGGGC
   heavy chain CDR2 nucleotide:
      ATCATCTTCAACGCTGGCGAGCGGACCGACTACGGCGACTCCGTGAAGGGA
   heavy chain CDR3 nucleotide:
      GTGTGGTGTGGCTCCTGGGTGGCTCGGTCC
SEQ ID NO.9: Full length amino acid sequence of the humanized single variable domain antibody hzF2 
EVQLVESGGGLVQPGGSLRLSCAASRDSDDGASCMGWFRQAPGKGLEGVAIIFNAGER
TDYGDSVKGRFTISRDNAKNTLYLQMNSLRAEDTAVYYCATVWCGSWVARSWGQGTLVT
VSSASEPKSSDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISKAK
GQPREPQVYTLPPSRDELTKNQVSLTCLVKGFYPSDIAVEWESNGQPENNYKTTPPVLDSDGSFF
LYSKLTVDKSRWQQGNVFSCSVMHEALHNHYTQKSLSLSPG
 wherein:
   heavy chain CDR1 amino acid: RDSDDGASCMG
   heavy chain CDR2 amino acid: IIFNAGERTDYGDSVKG
   heavy chain CDR3 amino acid: VWCGSWVARS
SEQ ID NO. 10: Full length nucleotide sequence of the humanized single variable domain antibody hzF2 
GAGGTGCAGCTGGTGGAGTCTGGAGGTGGCCTGGTGCAGCCTGGAGGCTCCCTGA
GGCTGTCCTGCGCTGCCTCTCGGGACTCCGACGACGGAGCCTCCTGCATGGGCTGGTTC
AGGCAGGCTCCTGGCAAGGGCCTGGAGGGAGTGGCCATCATCTTCAACGCTGGCGAGC
GGACCGACTACGGCGACTCCGTGAAGGGACGGTTCACCATCTCCAGGGACAACGCCAA
GAACACCCTGTACCTGCAGATGAACTCCCTGAGAGCCGAGGACACAGCCGTGTACTACT
GCGCTACCGTGTGGTGTGGCTCCTGGGTGGCTCGGTCCTGGGGACAGGGCACCCTGGTG
ACCGTGTCCTCCGCTAGCGAGCCCAAATCTAGCGACAAAACTCACACATGCCCACCGTGCCC
AGCACCTGAACTCCTGGGGGGACCGTCAGTCTTCCTCTTCCCCCCAAAACCCAAGGACACCC
TCATGATCTCCCGGACCCCTGAGGTCACATGCGTGGTGGTGGACGTGAGCCACGAAGACCC
TGAGGTCAAGTTCAACTGGTACGTGGACGGCGTGGAGGTGCATAATGCCAAGACAAAGCCGC
GGGAGGAGCAGTACAACAGCACGTACCGTGTGGTCAGCGTCCTCACCGTCCTGCACCAGGA
CTGGCTGAATGGCAAGGAGTACAAGTGCAAGGTCTCCAACAAAGCCCTCCCAGCCCCCATCG
AGAAAACCATCTCCAAAGCCAAAGGGCAGCCCCGAGAACCACAGGTGTACACCCTGCCTCCA
TCTCGGGATGAGCTGACCAAGAACCAGGTCAGCCTGACCTGCCTGGTCAAAGGCTTCTATCC
CAGCGACATCGCCGTGGAGTGGGAGAGCAATGGGCAGCCGGAGAACAACTACAAGACCACG
CCTCCCGTGCTGGACTCCGACGGCTCCTTCTTCCTCTATAGCAAGCTCACCGTGGACAAGAG
CAGGTGGCAGCAGGGGAACGTCTTCTCATGCTCCGTGATGCATGAGGCTCTGCACAACCACT
ACACGCAGAAGAGCCTCTCCCTGTCCCCGGGT
 wherein:
   heavy chain CDR1 nucleotide:
      CGGGACTCCGACGACGGAGCCTCCTGCATGGGC
   heavy chain CDR2 nucleotide:
      ATCATCTTCAACGCTGGCGAGCGGACCGACTACGGCGACTCCGTGAAGGGA
   heavy chain CDR3 nucleotide:
      GTGTGGTGTGGCTCCTGGGTGGCTCGGTCC
SEQ ID NO. 11: Variable region amino acid sequence of the humanized single variable domain antibody hzF2-m1 
EVQLVESGGGLVQPGGSLRLSCAASRDSDDGASSMGWFRQAPGKGLEGVAIIFNAGE
RTDYGDSVKGRFTISRDNAKNTLYLQMNSLRAEDTAVYYCATVWSGSWVARSWGQGTL
VTVSS
SEQ ID NO. 12: Variable region amino acid sequence of the humanized single variable domain antibody hzF2-m2 
EVQLVESGGGLVQPGGSLRLSCAASGDSDDGASCMGWFRQAPGKGLEGVAIIFNAGE
RTDYGDSVKGRFTISRDNAKNTLYLQMNSLRAEDTAVYYCATVWCGSWVARSWGQGTL
VTVSS
SEQ ID NO. 13: Variable region amino acid sequence of the humanized single variable domain antibody hzF2-m3 
EVQLVESGGGLVQPGGSLRLSCAASRDSSSGASCMGWFRQAPGKGLEGVAIIFNAGER
TDYGDSVKGRFTISRDNAKNTLYLQMNSLRAEDTAVYYCATVWCGSWVARSWGQGTLV
TVSS
SEQ ID NO. 14: Variable region amino acid sequence of the humanized single variable domain antibody hzF2-m4 
EVQLVESGGGLVQPGGSLRLSCAASRDSNDGASCMGWFRQAPGKGLEGVAIIFNAGE
RTDYGDSVKGRFTISRDNAKNTLYLQMNSLRAEDTAVYYCATVWCGSWVARSWGQGTL
VTVSS
SEQ ID NO. 15: Variable region amino acid sequence of the humanized single variable domain antibody hzF2-m5 
EVQLVESGGGLVQPGGSLRLSCAASRDSDDAASCMGWFRQAPGKGLEGVAIIFNAGE
RTDYGDSVKGRFTISRDNAKNTLYLQMNSLRAEDTAVYYCATVWCGSWVARSWGQGTL
VTVSS
SEQ ID NO. 16: Variable region amino acid sequence of the humanized single variable domain antibody hzF2-m6 
EVQLVESGGGLVQPGGSLRLSCAASRDSDEGASCMGWFRQAPGKGLEGVAIIFNAGE
RTDYGDSVKGRFTISRDNAKNTLYLQMNSLRAEDTAVYYCATVWCGSWVARSWGQGTL
VTVSS
SEQ ID NO. 17: Variable region amino acid sequence of the humanized single variable domain antibody hzF2-m7 
EVQLVESGGGLVQPGGSLRLSCAASRDSDDSASCMGWFRQAPGKGLEGVAIIFNAGE
RTDYGDSVKGRFTISRDNAKNTLYLQMNSLRAEDTAVYYCATVWCGSWVARSWGQGTL
VTVSS
SEQ ID NO. 18: Variable region amino acid sequence of the humanized single variable domain antibody hzF2-m8 
EVQLVESGGGLVQPGGSLRLSCAASRDSDDGASCMGWFRQAPGKEREGVAIIFNVGE
RTDYGDSVKGRFTISRDNAKNTLYLQMNSLRAEDTAVYYCATVWCGSWVARSWGQGTL
VTVSS
SEQ ID NO. 19: Variable region amino acid sequence of the humanized single variable domain antibody hzF2-m9 
EVQLVESGGGLVQPGGSLRLSCAASRDSDEGASCMGWFRQAPGKEREGVAIIFNAGER
TDYGDSVKGRFTISRDNAKNTLYLQMNSLRAEDTAVYYCATVWCGSWVARSWGQGTLVT
VSS
SEQ ID NO.20: Variable region amino acid sequence of the humanized single variable domain antibody hzF2-m10 
EVQLVESGGGLVQPGGSLRLSCAASRDSDDGASCMGWFRQAPGKEREGVAIIFNAGER
TDYGDSVKGRFTISRDNAKNTLYLQMNSLRAEDTAVYYCATVYCGSWVARSWGQGTLVTV
SS
SEQ ID NO.21: Variable region amino acid sequence of the humanized single variable domain antibody hzF2-m1 1 
EVQLVESGGGLVQPGGSLRLSCAASRDSDDGASCMGWFRQAPGKEREGVAIIFNAGER
TDYGDSVKGRFTISRDNAKNTLYLQMNSLRAEDTAVYYCATVYCGSYVARSWGQGTLVTV
SS
SEQ ID NO.22: Variable region amino acid sequence of the humanized single variable domain antibody hzF2-m12 
EVQLVESGGGLVQPGGSLRLSCAASRDSDEGASCMGWFRQAPGKEREGVAIIFNVGER
TDYGDSVKGRFTISRDNAKNTLYLQMNSLRAEDTAVYYCATVWCGSWVARSWGQGTLVT
VSS
SEQ ID NO.23: Variable region amino acid sequence of the humanized single variable domain antibody hzF2-m13 
EVQLVESGGGLVQPGGSLRLSCAASRDSDDGASCMGWFRQAPGKEREGVAIIFNAGER
TDYGDSVKGRFTISRDNAKNTLYLQMNSLRAEDTAVYYCATVFCGSFVARSWGQGTLVTVS
S
SEQ ID NO.24: Variable region amino acid sequence of the humanized single variable domain antibody hzF2-m14 EVQLVESGGGLVQPGGSLRLSCAASRDSDEGASCMGWFRQAPGKEREGVAIIFNVGERT DYGDSVKGRFTISRDNAKNTLYLQMNSLRAEDTAVYYCATVYCGSYVARSWGQGTLVTVSS
SEQ ID NO.25: Variable region amino acid sequence of the humanized single variable domain antibody hzF2-m15 
EVQLVESGGGLVQPGGSLRLSCAASRDSDEGASCMGWFRQAPGKEREGVAIIFNVGERT
DYGDSVKGRFTISRDNAKNTLYLQMNSLRAEDTAVYYCATVFCGSYVARSWGQGTLVTVSS
SEQ ID NO.26: Variable region amino acid sequence of the humanized single variable domain antibody hzF2-m16 
EVQLVESGGGLVQPGGSLRLSCAASRDSDEGASCMGWFRQAPGKEREGVAIIFNVGERT
DYGDSVKGRFTISRDNAKNTLYLQMNSLRAEDTAVYYCATVFCGSFVARSWGQGTLVTVSS
SEQ ID NO.27: Variable region nucleotide sequence of the humanized single variable domain antibody hzF2-m1 
GAGGTGCAGCTGGTGGAGTCTGGAGGTGGCCTGGTGCAGCCTGGAGGCTCCCTGA
GGCTGTCCTGCGCTGCCTCTCGGGACTCCGACGACGGAGCCTCCAGCATGGGCTGGTTC
AGGCAGGCTCCTGGCAAGGGCCTGGAGGGAGTGGCCATCATCTTCAACGCTGGCGAGC
GGACCGACTACGGCGACTCCGTGAAGGGACGGTTCACCATCTCCAGGGACAACGCCAA
GAACACCCTGTACCTGCAGATGAACTCCCTGAGAGCCGAGGACACAGCCGTGTACTACT
GCGCTACCGTGTGGTCCGGCTCCTGGGTGGCTCGGTCCTGGGGACAGGGCACCCTGGTG
ACCGTGTCCTCC
SEQ ID NO.28: Variable region nucleotide sequence of the humanized single variable domain antibody hzF2-m2 
GAGGTGCAGCTGGTGGAGTCTGGAGGTGGCCTGGTGCAGCCTGGAGGCTCCCTGA
GGCTGTCCTGCGCTGCCTCTGGAGACTCCGACGACGGAGCCTCCTGCATGGGCTGGTTC
AGGCAGGCTCCTGGCAAGGGCCTGGAGGGAGTGGCCATCATCTTCAACGCTGGCGAGC
GGACCGACTACGGCGACTCCGTGAAGGGACGGTTCACCATCTCCAGGGACAACGCCAA
GAACACCCTGTACCTGCAGATGAACTCCCTGAGAGCCGAGGACACAGCCGTGTACTACT
GCGCTACCGTGTGGTGTGGCTCCTGGGTGGCTCGGTCCTGGGGACAGGGCACCCTGGTG
ACCGTGTCCTCC
SEQ ID NO.29: Variable region nucleotide sequence of the humanized single variable domain antibody hzF2-m3 
GAGGTGCAGCTGGTGGAGTCTGGAGGTGGCCTGGTGCAGCCTGGAGGCTCCCTGA
GGCTGTCCTGCGCTGCCTCTCGGGACTCCAGCAGCGGAGCCTCCTGCATGGGCTGGTTC
AGGCAGGCTCCTGGCAAGGGCCTGGAGGGAGTGGCCATCATCTTCAACGCTGGCGAGC
GGACCGACTACGGCGACTCCGTGAAGGGACGGTTCACCATCTCCAGGGACAACGCCAA
 
GAACACCCTGTACCTGCAGATGAACTCCCTGAGAGCCGAGGACACAGCCGTGTACTACT
GCGCTACCGTGTGGTGTGGCTCCTGGGTGGCTCGGTCCTGGGGACAGGGCACCCTGGTG
ACCGTGTCCTCC
SEQ ID NO.30: Variable region nucleotide sequence of the humanized single variable domain antibody hzF2-m4 
GAGGTGCAGCTGGTGGAGTCTGGAGGTGGCCTGGTGCAGCCTGGAGGCTCCCTGA
GGCTGTCCTGCGCTGCCTCTCGGGACTCCAACGACGGAGCCTCCTGCATGGGCTGGTTC
AGGCAGGCTCCTGGCAAGGGCCTGGAGGGAGTGGCCATCATCTTCAACGCTGGCGAGC
GGACCGACTACGGCGACTCCGTGAAGGGACGGTTCACCATCTCCAGGGACAACGCCAA
GAACACCCTGTACCTGCAGATGAACTCCCTGAGAGCCGAGGACACAGCCGTGTACTACT
GCGCTACCGTGTGGTGTGGCTCCTGGGTGGCTCGGTCCTGGGGACAGGGCACCCTGGTG
ACCGTGTCCTCC
SEQ ID NO.31: Variable region nucleotide sequence of the humanized single variable domain antibody hzF2-m5 
GAGGTGCAGCTGGTGGAGTCTGGAGGTGGCCTGGTGCAGCCTGGAGGCTCCCTGA
GGCTGTCCTGCGCTGCCTCTCGGGACTCCGACGACGCTGCCTCCTGCATGGGCTGGTTC
AGGCAGGCTCCTGGCAAGGGCCTGGAGGGAGTGGCCATCATCTTCAACGCTGGCGAGC
GGACCGACTACGGCGACTCCGTGAAGGGACGGTTCACCATCTCCAGGGACAACGCCAA
GAACACCCTGTACCTGCAGATGAACTCCCTGAGAGCCGAGGACACAGCCGTGTACTACT
GCGCTACCGTGTGGTGTGGCTCCTGGGTGGCTCGGTCCTGGGGACAGGGCACCCTGGTG
ACCGTGTCCTCC
SEQ ID NO.32: Variable region nucleotide sequence of the humanized single variable domain antibody hzF2-m6 
GAGGTGCAGCTGGTGGAGTCTGGAGGTGGCCTGGTGCAGCCTGGAGGCTCCCTGA
GGCTGTCCTGCGCTGCCTCTCGGGACTCCGACGAGGGAGCCTCCTGCATGGGCTGGTTC
AGGCAGGCTCCTGGCAAGGGCCTGGAGGGAGTGGCCATCATCTTCAACGCTGGCGAGC
GGACCGACTACGGCGACTCCGTGAAGGGACGGTTCACCATCTCCAGGGACAACGCCAA
GAACACCCTGTACCTGCAGATGAACTCCCTGAGAGCCGAGGACACAGCCGTGTACTACT
GCGCTACCGTGTGGTGTGGCTCCTGGGTGGCTCGGTCCTGGGGACAGGGCACCCTGGTG
ACCGTGTCCTCC
SEQ ID NO.33: Variable region nucleotide sequence of the humanized single variable domain antibody hzF2-m7 
GAGGTGCAGCTGGTGGAGTCTGGAGGTGGCCTGGTGCAGCCTGGAGGCTCCCTGA
GGCTGTCCTGCGCTGCCTCTCGGGACTCCGACGACTCTGCCTCCTGCATGGGCTGGTTC
 
AGGCAGGCTCCTGGCAAGGGCCTGGAGGGAGTGGCCATCATCTTCAACGCTGGCGAGC
GGACCGACTACGGCGACTCCGTGAAGGGACGGTTCACCATCTCCAGGGACAACGCCAA
GAACACCCTGTACCTGCAGATGAACTCCCTGAGAGCCGAGGACACAGCCGTGTACTACT
GCGCTACCGTGTGGTGTGGCTCCTGGGTGGCTCGGTCCTGGGGACAGGGCACCCTGGTG
ACCGTGTCCTCC
SEQ ID NO.34: Variable region nucleotide sequence of the humanized single variable domain antibody hzF2-m8 
GAGGTGCAGCTGGTGGAGTCTGGAGGTGGCCTGGTGCAGCCTGGAGGCTCCCTGA
GGCTGTCCTGCGCTGCCTCTCGGGACTCCGACGACGGAGCCTCCTGCATGGGCTGGTTC
AGGCAGGCTCCTGGCAAGGAGAGAGAGGGAGTGGCCATCATCTTCAACGTGGGCGAGC
GGACCGACTACGGCGACTCCGTGAAGGGACGGTTCACCATCTCCAGGGACAACGCCAA
GAACACCCTGTACCTGCAGATGAACTCCCTGAGAGCCGAGGACACAGCCGTGTACTACT
GCGCTACCGTGTGGTGTGGCTCCTGGGTGGCTCGGTCCTGGGGACAGGGCACCCTGGTG
ACCGTGTCCTCC
SEQ ID NO.35: Variable region nucleotide sequence of the humanized single variable domain antibody hzF2-m9 
GAGGTGCAGCTGGTGGAGTCTGGAGGTGGCCTGGTGCAGCCTGGAGGCTCCCTGA
GGCTGTCCTGCGCTGCCTCTCGGGACTCCGACGAGGGAGCCTCCTGCATGGGCTGGTTC
AGGCAGGCTCCTGGCAAGGAGAGAGAGGGAGTGGCCATCATCTTCAACGCTGGCGAGC
GGACCGACTACGGCGACTCCGTGAAGGGACGGTTCACCATCTCCAGGGACAACGCCAA
GAACACCCTGTACCTGCAGATGAACTCCCTGAGAGCCGAGGACACAGCCGTGTACTACT
GCGCTACCGTGTGGTGTGGCTCCTGGGTGGCTCGGTCCTGGGGACAGGGCACCCTGGTG
ACCGTGTCCTCC
SEQ ID NO.36: Variable region nucleotide sequence of the humanized single variable domain antibody hzF2-m10 
GAGGTGCAGCTGGTGGAGTCTGGAGGTGGCCTGGTGCAGCCTGGAGGCTCCCTGA
GGCTGTCCTGCGCTGCCTCTCGGGACTCCGACGACGGAGCCTCCTGCATGGGCTGGTTC
AGGCAGGCTCCTGGCAAGGAGAGAGAGGGAGTGGCCATCATCTTCAACGCTGGCGAGC
GGACCGACTACGGCGACTCCGTGAAGGGACGGTTCACCATCTCCAGGGACAACGCCAA
GAACACCCTGTACCTGCAGATGAACTCCCTGAGAGCCGAGGACACAGCCGTGTACTACT
GCGCTACCGTGTACTGTGGCTCCTGGGTGGCTCGGTCCTGGGGACAGGGCACCCTGGTG
ACCGTGTCCTCC
SEQ ID NO.37: Variable region nucleotide sequence of the humanized single variable domain antibody hzF2-m11 
GAGGTGCAGCTGGTGGAGTCTGGAGGTGGCCTGGTGCAGCCTGGAGGCTCCCTGA
GGCTGTCCTGCGCTGCCTCTCGGGACTCCGACGACGGAGCCTCCTGCATGGGCTGGTTC
AGGCAGGCTCCTGGCAAGGAGAGAGAGGGAGTGGCCATCATCTTCAACGCTGGCGAGC
GGACCGACTACGGCGACTCCGTGAAGGGACGGTTCACCATCTCCAGGGACAACGCCAA
GAACACCCTGTACCTGCAGATGAACTCCCTGAGAGCCGAGGACACAGCCGTGTACTACT
GCGCTACCGTGTACTGTGGCTCCTACGTGGCTCGGTCCTGGGGACAGGGCACCCTGGTG
ACCGTGTCCTCC
SEQ ID NO.38: Variable region nucleotide sequence of the humanized single variable domain antibody hzF2-m12 
GAGGTGCAGCTGGTGGAGTCTGGAGGTGGCCTGGTGCAGCCTGGAGGCTCCCTGA
GGCTGTCCTGCGCTGCCTCTCGGGACTCCGACGAGGGAGCCTCCTGCATGGGCTGGTTC
AGGCAGGCTCCTGGCAAGGAGAGAGAGGGAGTGGCCATCATCTTCAACGTGGGCGAGC
GGACCGACTACGGCGACTCCGTGAAGGGACGGTTCACCATCTCCAGGGACAACGCCAA
GAACACCCTGTACCTGCAGATGAACTCCCTGAGAGCCGAGGACACAGCCGTGTACTACT
GCGCTACCGTGTGGTGTGGCTCCTGGGTGGCTCGGTCCTGGGGACAGGGCACCCTGGTG
ACCGTGTCCTCC
SEQ ID NO.39: Variable region nucleotide sequence of the humanized single variable domain antibody hzF2-m13 
GAGGTGCAGCTGGTGGAGTCTGGAGGTGGCCTGGTGCAGCCTGGAGGCTCCCTGA
GGCTGTCCTGCGCTGCCTCTCGGGACTCCGACGACGGAGCCTCCTGCATGGGCTGGTTC
AGGCAGGCTCCTGGCAAGGAGAGAGAGGGAGTGGCCATCATCTTCAACGCTGGCGAGC
GGACCGACTACGGCGACTCCGTGAAGGGACGGTTCACCATCTCCAGGGACAACGCCAA
GAACACCCTGTACCTGCAGATGAACTCCCTGAGAGCCGAGGACACAGCCGTGTACTACT
GCGCTACCGTGTTCTGTGGCTCCTTCGTGGCTCGGTCCTGGGGACAGGGCACCCTGGTG
ACCGTGTCCTCC
SEQ ID NO.40: Variable region nucleotide sequence of the humanized single variable domain antibody hzF2-m14 
GAGGTGCAGCTGGTGGAGTCTGGAGGTGGCCTGGTGCAGCCTGGAGGCTCCCTGA
GGCTGTCCTGCGCTGCCTCTCGGGACTCCGACGAGGGAGCCTCCTGCATGGGCTGGTTC
AGGCAGGCTCCTGGCAAGGAGAGAGAGGGAGTGGCCATCATCTTCAACGTGGGCGAGC
GGACCGACTACGGCGACTCCGTGAAGGGACGGTTCACCATCTCCAGGGACAACGCCAA
GAACACCCTGTACCTGCAGATGAACTCCCTGAGAGCCGAGGACACAGCCGTGTACTACT
GCGCTACCGTGTACTGTGGCTCCTACGTGGCTCGGTCCTGGGGACAGGGCACCCTGGTG
ACCGTGTCCTCC
SEQ ID NO.41: Variable region nucleotide sequence of the humanized single variable domain antibody hzF2-m15 
GAGGTGCAGCTGGTGGAGTCTGGAGGTGGCCTGGTGCAGCCTGGAGGCTCCCTGA
GGCTGTCCTGCGCTGCCTCTCGGGACTCCGACGAGGGAGCCTCCTGCATGGGCTGGTTC
AGGCAGGCTCCTGGCAAGGAGAGAGAGGGAGTGGCCATCATCTTCAACGTGGGCGAGC
GGACCGACTACGGCGACTCCGTGAAGGGACGGTTCACCATCTCCAGGGACAACGCCAA
GAACACCCTGTACCTGCAGATGAACTCCCTGAGAGCCGAGGACACAGCCGTGTACTACT
GCGCTACCGTGTTCTGTGGCTCCTACGTGGCTCGGTCCTGGGGACAGGGCACCCTGGTG
ACCGTGTCCTCC
SEQ ID NO.42: Variable region nucleotide sequence of the humanized single variable domain antibody hzF2-m16

GAGGTGCAGCTGGTGGAGTCTGGAGGTGGCCTGGTGCAGCCTGGAGGCTCCCTGA
GGCTGTCCTGCGCTGCCTCTCGGGACTCCGACGAGGGAGCCTCCTGCATGGGCTGGTTC
AGGCAGGCTCCTGGCAAGGAGAGAGAGGGAGTGGCCATCATCTTCAACGTGGGCGAGC
GGACCGACTACGGCGACTCCGTGAAGGGACGGTTCACCATCTCCAGGGACAACGCCAA
GAACACCCTGTACCTGCAGATGAACTCCCTGAGAGCCGAGGACACAGCCGTGTACTACT
GCGCTACCGTGTTCTGTGGCTCCTTCGTGGCTCGGTCCTGGGGACAGGGCACCCTGGTG
ACCGTGTCCTCC

The above descriptions are only preferred embodiments of the present invention, but the protection scopes of the present invention are not limited to these. Any changes or variations that can be readily conceived by those skilled in the art and within the technical scopes disclosed by the present invention shall be covered within the protection scopes of the present invention. Therefore, the protection scopes of the present invention should be those claimed in the claims.

## Claims

1. An anti-PD-L1 single variable domain antibody, **characterized in that** CDR1-CDR3 in a variable region of the single variable domain antibody are shown as SEQ ID NOs: 43-45 respectively.

2. The anti-PD-L1 single variable domain antibody according to claim 1, **characterized in that** the single variable domain antibody has no constant region or has 1-3 heavy chain constant regions.

3. The anti-PD-L1 single variable domain antibody according to any one of claims 1-2, **characterized in that** the amino acid sequence of the variable region of the single variable domain antibody is shown as SEQ ID NO: 1.

4. An anti-PD-L1 single variable domain antibody, **characterized in that** the single variable domain antibody is a human-camel chimeric single variable domain antibody, comprising the variable region of the single variable domain antibody according to any one of claims 1-3 and human heavy chain constant regions.

5. The anti-PD-L1 single variable domain antibody according to claim 4, **characterized in that** the chimeric single variable domain antibody has the amino acid sequence shown as SEQ ID NO:3.

6. An anti-PD-L1 single variable domain antibody, **characterized in that** the single variable domain antibody is humanized, and the variable region of the single variable domain antibody is obtained by humanizing the variable region of the single variable domain according to any one of claims 1-3.

7. The anti-PD-L1 single variable domain antibody according to claim 6, **characterized in that** the variable region of the single variable domain antibody has the amino acid sequence shown as SEQ ID NO:7.

8. The anti-PD-L1 single variable domain antibody according to claim 6 or 7, **characterized in that** the single variable domain antibody has the amino acid sequence shown as SEQ ID NO:9.

9. An anti-PD-L1 single variable domain antibody, **characterized in that** the single variable domain antibody is a mutated anti-PD-L1 humanized single variable domain antibody, which is produced by mutating CDRs in the variable region of anti-PD-L1 single variable domain antibody according to any of claims 6-8 by 1, 2, 3 or 4 amino acid residues; and the mutated anti-PD-L1 humanized single variable domain antibody at least partially retains the specific binding ability to PD-L 1.

10. The anti-PD-L1 single variable domain antibody according to claim 9, **characterized in that** the variable region of the single variable domain antibody is selected from the group consisting of SEQ ID NOs: 11-26.

11. A composition comprising one or more anti-PD-L1s selected from the group consisting of the anti-PD-L1 single variable domain antibodies according to any one of claims 1-10.

12. The composition according to claim 11, further comprising a pharmaceutically acceptable carrier, and used as a pharmaceutical composition, preferably the pharmaceutical composition is in the form of a liquid formulation, an injection formulation, or a powder injection formulation.

13. Use of an antibody or a fragment thereof for the manufacture of a medicament for the treatment of abnormal proliferative diseases, **characterized in that** the antibody is selected from the group consisting of the anti-PD-L1 single variable domain antibody according to any one of claims 1-10.

14. The use according to claim 13, **characterized in that** the abnormal proliferative diseases comprise tumors, preferably melanoma, non-small cell lung cancer, head and neck squamous carcinoma, kidney cancer, colon cancer and the like.

15. Use of an antibody or a fragment thereof, **characterized in that** the antibody is selected from the group consisting of the anti-PD-L1 single variable domain antibody according to any one of claims 1-10, for the manufacture of a multispecific antibody or a targeted antibody-drug.

16. A polynucleotide encoding the anti-PD-L1 single variable domain antibody according to any one of claims 1-10.

17. A vector comprising the polynucleotide according to claim 16.

18. A host cell comprising the polynucleotide according to claim 16 or the vector according to claim 17.

19. A method for preparing an anti-PD-L1 single variable domain antibody, comprising the steps of:
(1) Culturing the host cell according to claim 18 under conditions suitable for expressing the recombinant anti-PD-L1 single variable domain antibody;
(2) Isolating and purifying the anti-PD-L1 single variable domain antibody from the cell culture.

20. A method for preventing or treating abnormal proliferative diseases, **characterized in that** administering to a subject in need thereof an effective amount of the anti-PD-L1 single variable domain antibody according to any one of claims 1-10, the composition according to any one of claims 11-12, the multispecific antibody or the targeted antibody-drug according to claim 15.

21. A method for diagnosing or evaluating the development and progress of abnormal proliferative diseases in a subject, **characterized in that** contacting a sample from the subject to be detected with the anti-PD-L1 single variable domain antibody according to any one of claims 1-10, the composition according to any one of claims 11-12, the multispecific antibody or the targeted antibody-drug according to claim 15.

22. A method for predicting or evaluating the therapeutic effect of a PD-1/PD-L1 antagonist on a subject suffering from an abnormal proliferative disease, **characterized in that** detecting the expression status of PD-L1 in the subject by using an agent selected from the group consisting of the anti-PD-L1 single variable domain antibody according to any one of claims 1-10, the composition according to any one of claims 11-12, the multispecific antibody or the targeted antibody-drug according to claim 15.

23. The method according to any one of claims 20-22, **characterized in that** the abnormal proliferative diseases comprises tumors, particularly tumors associated with PD-1/PD-L1 signaling pathway.

24. The method according to claim 24, wherein the tumors comprise melanoma, non-small cell lung cancer, head and neck squamous carcinoma, kidney cancer, colon cancer and the like.
